# EUROPÄISCHE PATENTSCHRIFT

(11) **EP 1 896 497 B1**
(45) Veröffentlichungstag und Bekanntmachung des Hinweises auf die Patenterteilung: **12.07.2017**
(21) Anmeldenummer: 06762214.2
(22) Anmeldetag: 27.06.2006
(51) Int. Cl.: C07K 14/47

(54) **VERFAHREN ZUR DIAGNOSE VON RHEUMATISCHEN ERKRANKUNGEN**
METHOD FOR DIAGNOSING RHEUMATIC DISEASES
PROCEDE POUR LE DIAGNOSTIC DE MALADIES RHUMATISMALES

(30) Priorität: 27.06.2005 DE 102005029845
(43) Veröffentlichungstag der Anmeldung: 12.03.2008
(73) Patentinhaber: Orgentec Diagnostika GmbH, 55129 Mainz (DE)
(72) Erfinder: BANG, Holger, 65795 Hattersheim (DE); BERG, Wigbert, 55131 Mainz (DE)
(74) Vertreter: Weickmann & Weickmann PartmbB
(86) Internationale Anmeldenummer: PCT/EP2006/006205
(87) Internationale Veröffentlichungsnummer: WO 2007/000320

(56) Entgegenhaltungen:
- WO-A-00/47193
- WO-A-00/74662
- VOSSENAAR E R ET AL: "Anti-CCP antibodies, a highly specific marker for (early) rheumatoid arthritis" CLINICAL AND APPLIED IMMUNOLOGY REVIEWS 2004 UNITED STATES, Bd. 4, Nr. 4, 2004, Seiten 239-262, XP002407634 ISSN: 1529-1049
- HAQQI T M ET AL: "PREVENTION OF COLLAGEN-INDUCED ARTHRITIS IN MICE BY A POLYPHENOLIC FRACTION FROM GREEN TEA" PROCEEDINGS OF THE NATIONAL ACADEMY OF SCIENCES OF USA, NATIONAL ACADEMY OF SCIENCE, WASHINGTON, DC, US, Bd. 96, Nr. 8, 13. April 1999 (1999-04-13), Seiten 4524-4529, XP000961089 ISSN: 0027-8424
- ADCOCKS C ET AL: "CATECHINS FROM GREEN TEA (CAMELLIA SINENSIS) INHIBIT BOVINE AND HUMAN CARTILAGE PROTEOGLYCAN AND TPYE II COLLAGEN DEGRADATION IN VITRO" JOURNAL OF NUTRITION, WISTAR INSTITUTE OF ANATOMY AND BIOLOGY, PHILADELPHIA, PA,, US, Bd. 132, Nr. 3, März 2002 (2002-03), Seiten 341-346, XP009041556 ISSN: 0022-3166
- DATABASE EMBL 5. Mai 2005 (2005-05-05), "vimentin protein" XP002407716 gefunden im EBI Database accession no. ADY26865
- BOEKEL VAN M A M ET AL: "AUTOANTIBODY SYSTEMS IN RHEUMATOID ARTHRITIS: SPECIFICITY, SENSITIVITY AND DIAGNOSTIC VALUE" ARTHRITIS RESEARCH, CURRENT SCIENCE, LONDON,, GB, Bd. 4, Nr. 2, 2002, Seiten 87-93, XP009012450 ISSN: 1465-9905
- PINHEIRO GERALDO CASTELAR ET AL: "Anti-cyclic citrullinated peptide antibodies in advanced rheumatoid arthritis." ANNALS OF INTERNAL MEDICINE. 5 AUG 2003, Bd. 139, Nr. 3, 5. August 2003 (2003-08-05), Seiten 234-235, XP002407635 ISSN: 1539-3704
- SCHELLEKENS G A ET AL: "CITRULLINE IS AN ESSENTIAL CONSTITUENT OF ANTIGENIC DETERMINANTS RECOGNIZED BY RHEUMATOID ARTHRITIS-SPECIFIC AUTOANTIBODIES" JOURNAL OF CLINICAL INVESTIGATION, NEW YORK, NY, US, Bd. 101, Nr. 1, Januar 1998 (1998-01), Seiten 273-281, XP000749589 ISSN: 0021-9738
- HOFMANN I ET AL: "INTERFERENCE IN VIMENTIN ASSEMBLY IN-VITRO BY SYNTHETIC PEPTIDES DERIVED FROM THE VIMENTIN HEAD DOMAIN" JOURNAL OF CELL SCIENCE, Bd. 101, Nr. 3, 1992, Seiten 687-700, XP002407636 ISSN: 0021-9533
- VOSSENAAR ERIK R ET AL: "Rheumatoid arthritis specific anti-Sa antibodies target citrullinated vimentin" ARTHRITIS RESEARCH, CURRENT SCIENCE, LONDON,, GB, Bd. 6, Nr. 2, 5. Februar 2004 (2004-02-05), Seiten R142-R150, XP021011474 ISSN: 1465-9905
- DATABASE EMBL 29. Juli 2004 (2004-07-29), "Hypothetical protein BF0713, Bacteroides fragilis NCTC9343" XP002424393 gefunden im EBI Database accession no. YP_210416
- DATABASE EMBL 1. Oktober 2004 (2004-10-01), "Hypothetical protein BF0786, Bacteroides fargilis YCH46" XP002424394 gefunden im EBI Database accession no. YP_098071
- DATABASE UNIPROT [Online] 01 Februar 1996 'RecName: Full=Vimentin; Flags: Fragment;' Retrieved from EBI, accession no. UNIPROT:P48670 Database accession no. P48670

## Beschreibung

Die Erfindung betrifft Polypeptide, die mit Rheuma-assoziierten Autoantikörpern reagieren. Die Erfindung betrifft außerdem ein Diagnostikum, das eines dieser Polypeptide enthält, einen diagnostischen Kit, der dieses Diagnostikum enthält, sowie ein Verfahren zum *in vitro-*Nachweis von rheumatischen Erkrankungen. Des Weiteren betrifft die Erfindung ein Arzneimittel, das eines der Polypeptide enthält, sowie die Verwendung der Polypeptide zur Herstellung eines Arzneimittels zur Prophylaxe und/oder zur Behandlung von rheumatischen Erkrankungen.

Rheumatische Erkrankungen, insbesondere Schmerzen im Bereich der Gelenke und des Bewegungsapparates gehören zu den häufigsten Krankheiten in Deutschland. Ein Labortest, der es ermöglicht, diese Schmerzen einer harmlosen Muskelverspannung, einer Arthrose oder der häufigsten und schwersten der Erkrankungen, der Rheumatoiden Arthritis (RA), zuzuordnen, ist bisher nicht bekannt.

Die Rheumatoide Arthritis ist eine Autoimmunkrankeit, bei der die Abwehrmechanismen des menschlichen Körpers irrtümlich körpereigenen Gelenkknorpel für fremd und feindlich halten und diesen angreifen. Ungefähr 1 von 100 Menschen leidet in westeuropäischen Ländern an Rheumatoider Arthritis. In den ersten Monaten der Erkrankung schreitet diese sehr rasch voran.

Eine wesentliche Schlüsselstrategie in der modernen Rheumatologie ist daher der frühzeitige Einsatz von biologischen Arzneistoffen, die den Krankheitsverlauf modifizieren. Zahlreiche klinische Studien haben gezeigt, dass mit geeigneten Wirkstoffen, z.B. mit TNF-Antagonisten, sehr gute Therapieerfolge und Ansprechraten erzielt werden können, wenn diese bei Patienten bereits im Frühstadium eingesetzt werden. Rheumatologen versuchen, das schmale Zeitfenster zwischen dem Beginn der Krankheit und dem Auftreten von strukturellen Gelenkschäden zu nutzen. Bisher ist jedoch aus dem Stand der Technik kein zuverlässiger und sensitiver Nachweis der Rheumatoiden Arthritis in diesem Zeitfenster bekannt.

Die Diagnose der Rheumatoiden Arthritis erfolgt nach den Klassifikationskriterien des ACR (American College of Rheumatology). Gemäß den Kriterien des ACR ist der Rheumafaktor der bisher grundlegende serologische Indikator zur Diagnose der Rheumatoiden Arthritis (RA). Rheumafaktoren sind eine Teilgruppe von Immunglobulinen, die sich durch die immunologische Kreuzreaktion gegen die Fc-Region von Immunglobulin G (IgG) auszeichnen.

Das Vorhandensein eines Rheumafaktors ist jedoch nicht auf Erkrankungen des rheumatischen Formenkreises (differenzialdiagnostische Anhaltspunkte) beschränkt, man findet Rheumafaktoren auch im Serum von Patienten mit Infektionserkrankungen, Hyperglobulinanämien, lymphoproliferativen B-Zell-Erkrankungen und allgemein bei älteren Bevölkerungsschichten.

Im Allgemeinen werden erhöhte Konzentrationen von Rheumafaktoren mit einem schwereren Krankheitsverlauf assoziiert. Dabei korrelieren die Konzentrationen nicht mit dem Aktivitätsgrad und dem Therapieerfolg. Auf Basis der Konzentration von Rheumafaktoren kann keine sensitive und spezifische Prognose für den Beginn einer Rheumatoiden Arthritis getroffen werden. Gesunde Menschen haben eine erhöhte Rheumafaktorkonzentration ohne zu erkranken, Patienten ohne Rheumafaktoren haben dagegen eine sehr aggressive Form der Rheumatoiden Arthritis.

Andere serologische Marker, wie der anti-citrulline Antikörper (CCP) oder der initiale HAQ-Score, mit dem Fähigkeiten im Alltag beurteilt werden, oder das Röntgen- oder Computer-Tomografie (CT)-Bild geben bei der Frühform nur geringe Aufschlüsse und sind alleine nicht aussagekräftig genug, um beurteilen zu können, wie die Prognose des Patienten sein wird.

Zur Optimierung der bestehenden Klassifikationskriterien des ACR werden von der US-amerikanischen Rheumatologischen Fachgesellschaft sieben Klassifikationskriterien vorgeschlagen, die auf eine schlechte Prognose hindeuten:
1. Morgensteifigkeit der Gelenke von mehr als einer Stunde,
2. Arthritis an drei oder mehr Gelenken,
3. Gelenkentzündung von mindestens drei Gelenkregionen zur gleichen Zeit,
4. Handgelenke oder Fingergelenke sind ebenfalls betroffen,
5. bilaterale Druckschmerzhaftigkeit von Metacarpophalangeal-Gelenken,
6. Erosionen im Röntgenbild,
7. Nachweis spezieller Rheumafaktoren und Anti-perinukleäre Faktor-Positivität (APF).

Autoantikörper gegen den so genannten Anti-perinukleären Faktor wurden erstmals von Young et al. bei Patienten mit Rheumatoider Arthritis beschrieben (Young, B.J.J. et al., Antikeratin antibodies in rheumatoid arthritis, B.M.J., 2 (1979), 97-99). Aufgrund ihrer spezifischen Reaktion gegen das verhornte Epithel des Stratum corneum auf Rattenoesophagusschnitten wurde lange Zeit Keratin als das entsprechende Antigen angesehen (Vincent, C.H. et al.; High diagnostic value in rheumatoid arthritis of antibodies to the stratum corneum of rat oesophagus epithelium, so-called "antikeratin antibodies", Ann. Rheumat. Dis. 48 (1989), 712-722). Die Antikörper werden aus diesem Grund bis heute als AntiKeratin-Antikörper (AKA) bezeichnet (Vincent, C.H. et al, Natural IgG to Epidermal Cytokeratins vs IgG to the Stratum Corneum of the Rat Oesophagus Epithelium, so-called "Antikeratin Antibodies", in Rheumatoid Arthritis and other Rheumatic Diseases; J. of Autoimmunity 4 (1991), 493-505; Paimela, L. et al., Antikeratin antibodies: diagnostic and prognostic markers for early rheumatoid Arthritis, Ann. Rheumat. Dis., 51 (1992) 743-746).

Spätere Untersuchungen haben darüber hinaus gezeigt, dass AKA oder APF auch durch Anti-Filaggrin-Antikörper erkannt werden. Somit wurde das basische Protein Filaggrin als Zielantigen identifiziert. Das 40 kDa-Protein aggregiert Zytokeratinfilamente und hilft mit, die intrazelluläre Fasermatrix der verhornten Zellen zu bilden (Simon, M. et al., The Cytokeratin Filament-Aggregating Protein Filaggrin is the Target of the So-called "Antikeratin Antibodies", Autoantibodies Specific for Rheumatoid Arthritis, J. Clin. Invest., 92 (1993), 1387-93).

Da APF, AKA und Anti-Filaggrin-Antikörper enthaltende Seren in gleicher Weise reagieren, sind diese Antikörpersysteme scheinbar identisch. Anti-Filaggrin-Antikörper vom Typ IgG stellen mit einer Spezifität von über 99 % einen hochspezifischen Marker für die Rheumatoide Arthritis dar. Die Antikörper sind prinzipiell früh nachweisbar und gehen dem klinischen Krankheitsbild voraus. In mehreren Studien konnten positive Korrelationen zu Schwere und Aktivität der Krankheit gefunden werden. Anti-Filaggrin-Antikörper korrelieren nicht mit Alter, Geschlecht oder Krankheitsdauer. Sie können in ca. 34 % der Rheumafaktor-negativen Patienten nachgewiesen werden und stellen hier eine wertvolle diagnostische Hilfe dar.

Mit heute gebräuchlichen Methoden sind die Antikörper jedoch nur in ca. 40 % der Fälle im Serum zu finden.

Aufgabe der vorliegenden Erfindung war es daher, neue Polypeptide zum Nachweis von mit rheumatischen Erkrankungen assozierten, insbesondere mit Rheumatoider Arthritis assoziierten, Antikörpern bereitzustellen, die eine sensitive und spezifische Diagnose, eine Klassifizierung und eine Prognose von rheumatischen Erkrankungen, insbesondere von Schmerzen im Bereich der Gelenke und des Bewegungsapparates ermöglichen.

Bei der Analyse der Antikörper-Bindung an natives Vimentin, d.h. der APF-Positivität oder anti-Sa-Reaktivität (E.R. Vossenaar et al; Rheumatoid arthritis specific anti-Sa antibodies target citrullinated vimentin; Arthritis Res. Ther. 6(2), (2004), 142-150)), wurde nun gefunden, dass das als nicht immunologisch reaktiv aus dem Stand der Technik bekannte native Vimentin (C.A. Hitchon et al.; Immune features of seronegative and seropositive arthritis in early synovitis studies; Curr. Opin. Rheumatol. 14(4), (2002), 348-353) in Form von mutierten immunologisch reaktiven Varianten vorliegen. Diese Erkenntnis ist unerwartet, da nach dem bisherigen Stand der Technik davon ausgegangen wurde, dass Vimentin citrulliert sein muss, um immunologisch reaktiv zu sein. Diese Annahme konnte von uns widerlegt werden, indem durch differenzielle Immunaffinitäts-chromatographie immunologisch reaktive Vimentin-Varianten mit mutierten Sequenzen aus humanen Monocyten angereichert werden konnten. Diese mutierten Varianten von nativem Vimentin unterscheiden sich von nativem Vimentin durch das Vorhandensein zusätzlicher Arginin-Reste und gegebenenfalls weiterer Sequenzunterschiede. Sie reagieren mit humanen RA-assoziierten Antikörpern und weisen überraschenderweise eine höhere Spezifität und Sensitivität als die aus dem Stand der Technik bekannten citrullinierten Peptide auf.

Ein Gegenstand der Erfindung ist daher ein Polypeptid, wie in Anspruch 1 definiert, das von nativem Vimentin mit der SEQ ID No. 1 abgeleitet ist und das sich gegenüber der nativen Sequenz durch das Vorhandensein von mindestens einem zusätzlichen Arginin-Rest unterscheidet.

Die zusätzlichen Arginin-Reste sind durch Substitution anderer Aminosäurereste des nativen humanen Vimentins in die Sequenz eingefügt. Das Polypeptid weist in mindestens einer der Positionen 16, 17, 19, 41, 58, 59, 60, 68, 76, 140, 142, 147, 363, 406 oder 452 einen Arginin-Rest auf. Besonders bevorzugte Positionen sind 41, 58, 59, 60 und/oder 68. Beispielsweise weist das Polypeptid in mindestens einer, zwei, drei oder vier Positionen einen zusätzlichen Arginin-Rest auf.

In einer weiteren Ausführungsform weist das Polypeptid außerdem in mindestens einer der Positionen 3, 20, 33, 36, 37, 94, 165, 361, 399 oder 426 gegenüber der nativen Sequenz einen zusätzlichen Leucin-Rest auf, vorzugsweise an den Positionen 33, 36 und/oder 37. Beispielsweise weist das Polypeptid in mindestens einer, zwei, drei oder vier Positionen einen zusätzlichen Leucin-Rest auf.

In einer weiteren Ausführungsform weist das Polypeptid in mindestens einer der Positionen 21, 41, 43, 50, 54, 62, 64 oder 89, gegenüber der nativen Sequenz einen zusätzlichen Prolin-Rest auf, vorzugsweise an den Positionen 41, 43, 50, 54 62, und/oder 64. Beispielsweise weist das Polypeptid in mindestens einer, zwei, drei oder vier Positionen einen Prolin-Rest auf.

In einer weiteren Ausführungsform weist das Polypeptid in Position 24 gegenüber der nativen Sequenz einen zusätzlichen Threonin-Rest auf.

In einer weiteren Ausführungsform weist das Polypeptid in mindestens einer der Positionen 25, 39, 42, 49, 55 oder 67, gegenüber der nativen Sequenz einen zusätzlichen Tyrosin-Rest auf. Beispielsweise weist das Polypeptid in mindestens einer, zwei, drei oder vier Positionen einen Tyrosin-Rest auf.

In einer weiteren Ausführungsform liegt in dem Polypeptid mindestens ein Arginin-Rest als Citrullin-Rest vor, z.B. in mindestens einer der Positionen 4, 12, 23, 28, 36, 45, 50, 64, 71, 100, 320, 364 oder 378. Beispielsweise weist das Polypeptid in mindestens einer, zwei, drei oder vier der Positionen einen Citrullin-Rest auf. Andererseits kann das Polypeptid jedoch auch ein citrullinfreies Polypeptid sein.

Bevorzugte Beispiele für Muteine des humanen Vimentin haben eine Sequenz mit der SEQ ID No. 2, 3, 4, 5, 6, 7, 8 oder 9.

Ein weiterer Gegenstand der Erfindung ist ein Fragment eines der o.g. Polypeptide wie in Anspruch 9 definiert, das aus mindestens sechs Aminosäuren von nativem Vimentin mit der SEQ ID No. 1 besteht und das mindestens einen Bereich mit mindestens einem Arginin-Rest enthält und das eine Reaktivität gegen mit rheumatoiden Erkrankungen assoziierten Autoantikörpern zeigt. Vorzugsweise liegt das Fragment im Bereich der Positionen 10-145. Besonders bevorzugt liegt das Fragment im Bereich der Positionen 30-70. Ein bevorzugtes Beispiel eines Fragments ist das Fragment 51-65 (C2). Die Länge des Fragments beträgt mindestens 6, besonders bevorzugt mindestens 8 Aminosäuren bis zu 120, vorzugsweise bis zu 100 und besonders bevorzugt bis zu 50 Aminosäuren.

Ein weiterer hierin beschriebener Gegenstand ist eine Nukleinsäure, die für ein oben beschriebenes Polypeptid kodiert. Als Nukleinsäuren kommen z.B. DNA und RNA, insbesondere cDNA infrage. Die Nukleinsäuren können zur rekombinanten Herstellung der Polypeptide in übliche eukaryontische oder prokaryontische Vektoren einkloniert und in geeigneten Wirtszellen exprimiert werden.

Ein weiterer Gegenstand der Erfindung ist ein Diagnostikum, das ein oder mehrere der oben beschriebenen Polypeptide oder deren Fragmente enthält. Das Diagnostikum kann das Polypeptid oder das Fragment in freier oder trägergebundener Form enthalten.

Es ist als ausgesprochen überraschend zu bezeichnen, dass sich die erfindungsgemäßen Polypeptide als hochspezifische und hochsensitive Antigene für die Diagnostik von Antikörpern in Körperflüssigkeiten von Patienten mit rheumatischen Erkrankungen, insbesondere mit entzündlichen Erkrankungen der Gelenke und des Bewegungsapparates, besonders bevorzugt von Rheumatoider Arthritis, erweisen. Bevorzugte Körperflüssigkeiten im Sinne der Erfindung sind Blut, Serum oder Plasma, besonders bevorzugt ist Serum.

Das erfindungsgemäße Diagnostikum weist eine Reihe von Vorteilen auf. So können, da die Polypeptide mehrere Antikörperbindungsstellen enthalten, sowohl monomere als auch multimere Antikörper effizient gebunden werden. Ein weiterer Vorteil des mutierten Polypeptids ist, dass dieses die Bereitstellung eines Diagnostikums ermöglicht, das mit 99 % Spezifität und 85 % Sensitivität Patienten mit entzündlichen und chronischen Erkrankungen der Gelenke und des Bewegungsapparates, insbesondere mit Rheumatoider Arthritis, identifizieren kann.

Aus dem Stand der Technik ist bisher kein vergleichbar spezifisches oder sensitives Diagnostikum bekannt, das den Nachweis von rheumatischen Erkrankungen, insbesondere von Rheumatoider Arthritis unter Verwendung eines citrullinfreien Proteins oder Peptids ermöglicht (P. J. Utz, Death, autoantigen modifications, and tolerance; Arthritis Res., 2, (2000), 101-114).

Ein weiterer Gegenstand der Erfindung ist ein diagnostischer Kit zur Verwendung zum Nachweis rheumatischer Erkrankungen, insbesondere von Rheumatoider Arthritis, der ein oben beschriebenes Diagnostikum enthält. Daneben kann der diagnostische Kit übliche Bestandteile, wie Puffer, Lösungsmittel und/oder Markierungsgruppen, enthalten.

Als Träger kommen Makromoleküle, wie DNA, RNA, medizinisch verträgliche Polymere, wie beispielsweise Polyethylen, Poly D,L-Laktide, Poly D,L-Laktid-co-glykolide, synthetische Biopolymere, wie beispielsweise Polylysine und Dextrane, und Proteine, wie beispielsweise Serumalbumin und Hämocyanin, infrage. Bevorzugt werden Dextrane in einem so genannten "Hydrocoating-Beschichtungsverfahren" verwendet (Gregorius, K., Mouritsen, S. und Elsner, H.I., Hydrocoating: a new method for coupling biomolecules to solid phases, J. Immunol. Methods 12 (1995), 65-73).

Ein weiterer Gegenstand der Erfindung ist ein Verfahren zum *in vitro-*Nachweis von rheumatischen Erkrankungen, insbesondere von Rheumatoider Arthritis, bei dem die Konzentration von Autoantikörpern in einer Körperflüssigkeit bestimmt wird. Das Verfahren erlaubt die Stellung einer Diagnose, die Klassifizierung und/oder die Bewertung des Schweregrades der Erkrankung. Als Nachweisreagenz dient das oben beschriebene Diagnostikum oder der oben beschriebene diagnostische Kit.

In dem erfindungsgemäßen Verfahren können als Nachweismethoden alle auf dem Gebiet der Diagnostik üblichen Methoden, wie
(a) enzymologische Methoden,
(b) Lumineszenz-basierende Methoden oder
(c) radiochemische Methoden
verwendet werden.

Als bevorzugte Nachweismethoden kommen in dem erfindungsgemäßen Verfahren ein Radioimmunoassay, ein Chemolumineszenzimmunoassay, ein Immuno-Blot-Assay oder ein Enzymimmunoassay, z.B. ein ELISA, infrage.

In einer Ausführungsform des erfindungsgemäßen Verfahrens wird zu einem oben beschriebenen Polypeptid, das an einen Träger gebunden ist, als Probe die zu analysierende Körperflüssigkeit hinzugegeben. Nach Inkubation der Probe werden die ungebundenen Bestandteile weggewaschen. Die spezifische Bindung der zu detektierenden Autoantikörper an das Polypeptid wird durch einen Sekundärantikörper nachgewiesen, der eine Markierungsgruppe trägt.

Als Sekundärantikörper kommen in dem erfindungsgemäßen Verfahren beispielsweise Antikörper infrage, die gegen humane Antikörper, wie etwa IgG, IgM, IgA oder/und IgE, z.B. den Fc-Teil von humanem IgG, gerichtet sind.

Als Markierungsgruppen kommen in dem erfindungsgemäßen Verfahren beispielsweise ein Enzym, wie beispielsweise die Peroxidase oder die Alkalische Phosphatase, eine radioaktive Markierung oder eine lumineszierende Markierungsgruppe, wie beispielsweise Acridiniumverbindungen, infrage.

Alternativ kann auch ein kompetitiver Inhibitionstest mit den erfindungsgemäßen Polypeptiden durchgeführt werden, in dem die Bindung eines markierten Rheumatoiden Arthritis (RA)-Autoantikörpers in Gegenwart einer Probe inhibiert wird, sofern in der Probe ebenfalls RA-Autoantikörper vorhanden sind.

Die erfindungsgemäßen Peptide können auch als Mittel zur Prognose oder/und zur Verlaufskontrolle bei der Behandlung von rheumatischen Erkrankungen, insbesondere Rheumatoide Arthritis, dienen. Als prognostische Mittel sind hierbei Peptide aus dem Bereich von Position 30-65 oder/und von Position 55-70 bevorzugt.

Eine weitere Aufgabe der vorliegenden Erfindung war es, Arzneimittel bereitzustellen, die selektiv die Bildung von Autoimmunkomplexen im Zusammenhang mit rheumatischen Erkrankungen, insbesondere Entzündungsprozessen hemmen oder verhindern, besonders bevorzugt im entzündeten Gelenk, die aber keine allgemeine Blockierung der Produktion von Antikörpern bewirken.

Diese Aufgabe wird erfindungsgemäß gelöst durch ein Arzneimittel, das ein oben beschriebenes Polypeptid oder Fragment enthält und für Anwendungen in der Human- oder Veterinärmedizin geeignet ist. Das Arzneimittel kann z.B. zur extrakorporalen Behandlung von Körperflüssigkeiten, z.B. Blut oder Plasma, dienen, um darin enthaltene Autoantikörper mit Festphase-gebundenen Antikörpern abzufangen und die behandelte Körperflüssigkeit wieder in den Patienten zurückzuleiten.

Die erfindungsgemäßen Beispiele zeigen überraschenderweise, dass es sich bei dem erfindungsgemäßen Polypeptid um ein primär mit dem Krankheitsverlauf verbundenes Antigen handelt, oder sogar um das primär die Krankheit initiierende Protein. Durch selektives Entfernen von Antikörpern aus den Körperflüssigkeiten von Patienten kann der Krankheitsverlauf günstig beeinflusst werden.

Weiterhin können die Polypeptide oder deren Fragmente auch direkt in Form einer pharmazeutischen Zusammensetzung, die pharmazeutisch verträgliche Träger, Lösungsmittel und/oder Hilfsstoffe enthalten kann, verabreicht werden.

Die pharmazeutische Zusammensetzung kann in Form einer Tablette, einer Kapsel, einer Lösung, einer Suspension, eines Aerosols, eines Sprays (Nasen- oder Halsspray), eines Gels, eines Pflasters etc. verabreicht werden.

Das Arzneimittel kann nach allen bekannten Methoden verabreicht werden, wobei die orale und die intravenöse Verabreichung besonders bevorzugt sind.

Die Dosis kann je nach Art und Schwere der Erkrankung variieren und liegt üblicherweise im Bereich von 1 bis 2 000 mg/Tag, bevorzugt im Bereich von 10 bis 200 mg/Tag.

Des Weiteren können RA-Patienten durch die identifizierten kurzen, synthetischen Proteinepitope der oben beschriebenen Polypeptide (u.a. C2-Epitop) therapiert werden, die sich aus der Aminosäuresequenz des nativen Vimentins ableiten.

Die Analyse von B-Zell-Epitopen von RA-Patienten lieferte den überraschenden Befund, dass 91 % aller Patienten mit einem kurzen synthetischen, linearen Peptidepitop (C2-Epitop), des erfindungsgemäßen Polypeptids, interagieren.

Des Weiteren konnte durch Mausexperimente festgestellt werden, dass Mäuse nach einer solchen Behandlung Immunzellen bildeten, die erfolgreich die Teilung antigenspezifischer Immunzellen unterdrücken.

Eine Kontrolle des Verlaufs und des Erfolgs der Therapie von rheumatischen Erkrankungen, insbesondere einer Rheumatoiden Arthritis, ist nach den derzeit üblichen Diagnoseverfahren nur mittels der arbeits- und zeitaufwändigen Methode der Disease Activity Score (DAS) möglich. Bei dieser Methode werden die Anzahl der geschwollenen Gelenke, die Anzahl der schmerzhaften Gelenke, die Entzündungsparameter (BSR oder CRP) errechnet und die Befindlichkeit des Patienten auf einer visuellen Analog-Skala erfasst.

Diese vier Einzelkomponenten werden nach einer Formel zusammengezählt. Der resultierende Zahlenwert gibt relativ zuverlässig und objektiv Auskunft über die aktuelle Erkrankungsaktivität und die Qualität der Therapie beim Patienten.

Die erfindungsgemäßen Polypeptide ermöglichen es nunmehr, den Verlauf und den Erfolg der Therapie von rheumatoiden Erkrankungen, insbesondere von Rheumatoider Arthritis zu kontrollieren.

Beispielsweise konnte bei Patienten, die mit dem Polypeptid in Position 30 bis 65 kreuzreagierten, eine rasche und effektive Therapie beobachtet werden, die teilweise nach zwei Jahren mit einer vollständigen Heilung ihren positiven Abschluss fand.

Patienten mit einer geringen Krankheitsaktivität (DAS-Score im Mittel 2,8) konnten durch einen ELISA auf der Basis der erfindungsgemäßen Polypeptide identifiziert werden (durchschnittlich < 300 U/ml). Korrelierend zum Erfolg der Behandlung (DAS Score nach Behandlung kleiner 1,5) mit Sulfasalazin oder Cortison reduzierten sich der Antikörpertiter im Verlauf von 1-2 Jahren auf durchschnittlich 1/6 des Ausgangswertes.

Patienten mit einer schweren Rheumatoiden Arthritis (DAS-Score im Mittel 4,9) wiesen durchschnittlich einen Antikörpertiter von > 1.000 U/ml auf. Ohne dass sich der DAS Score durch die Behandlung mit Remicade und/oder Methrotrexat signifikant veränderte, reduzierte sich bei 50 % der Patienten die Menge an Antikörpern gegen die erfindungsgemäßen Polypeptide um ca. 30-50 %. Parallel zum veränderten Antikörpertiter wurde von Patienten eine wesentlich positivere Gesamteinschätzung der subjektiven Krankheitssituation bei der Anamnese zu Protokoll gegeben, d.h. das erfindungsgemäße Diagnostikum eignet sich auch zur Qualitätskontrolle der Therapie einer schweren Rheumatoiden Arthritis.

Ein wesentlicher Vorteil ist, dass in wenigen Minuten erstmals eine qualitativ und quantitativ zuverlässige Auskunft über die Art, den Verlauf und die Therapie der Rheumatoiden Arthritis gegeben werden kann.

Dieser überraschende Befund führte zu der Erkenntnis, dass die erfindungsgemäßen Polypeptide, therapeutisch verwendet werden können, weil durch deren Verwendung überraschenderweise eine Verminderung der bisherigen unerwünschten Arzneimittelwirkungen bei gleichbleibender Wirksamkeit zu erwarten ist.

Insbesondere ermöglichen die erfindungsgemäßen Polypeptide überraschenderweise die Bereitstellung eines neuartigen, bisher unbekannten Wirkprinzips zur Behandlung von rheumatischen Erkrankungen, insbesondere von Entzündungen. Das Wirkungspektrum der erfindungsgemäßen Polypeptide ist ein anderes als das bisher bekannter Hemmer von Entzündungen, da selektiv nur antikörperabhängige Mediatoren vermindert gebildet werden können. Ein weiterer Vorteil der erfindungsgemäßen Polypeptide ist, dass das Nebenwirkungspektrum voraussichtlich bedeutend geringer ist als das von aus dem Stand der Technik bekannten Arzneimitteln, da keine Kreuzreaktion mit Antikörpern von gesunden Blutspendern beobachtet werden konnte.

Das oben beschriebene erfindungsgemäße Diagnostikum und Therapeutikum kann daher für die Diagnostik, Prophylaxe oder Behandlung aller Erscheinungsformen angewendet werden, die auf Vimentin-abhängigen Prozessen beruhen.

Ein weiterer Gegenstand der vorliegenden Erfindung ist die Verwendung eines Polypeptids gemäß einer der Sequenzen SEQ ID NOs: 2-9 oder eines Fragments davon, bestehend aus mindestens sechs Aminosäuren, wobei das Fragment in mindestens einer der Positionen 16, 17, 19, 41, 58, 59, 60, 68, 76, 140, 142, 147, 363, 406 oder 452 der Sequenzen SEQ ID Nos: 2-9 einen Arginin-Rest umfasst und eine Reaktivität gegen Rheuma-assoziierte Antikörper zeigt zur Herstellung eines

Arzneimittels zur Prophylaxe und/oder zur Behandlung von rheumatischen Erkrankungen, insbesondere von Schmerzen in Gelenken und des Bewegungsapparates. Bezüglich der diagnostischen und pharmazeutischen Anwendungen wird auf die obigen Ausführungen zu den Vimentin-Analoga verwiesen.

Eine weitere Aufgabe der vorliegenden Erfindung war es, Substanzen zu finden, die selektiv die Bildung von Autoimmunkomplexen aus Rheuma-assoziierten Autoantikörpern mit Autoantigenen, insbesondere mit Vimentin, blockieren, d.h. nur die Reaktion der an der Pathogenese beteiligten Antikörpern verhindern, die die Antikörperproduktion und -reaktion aber nicht im Allgemeinen beeinflussen und die somit eine sensitive und spezifische Therapie oder Prophylaxe von Rheuma-assoziierten Erkrankungen von Schmerzen im Bereich der Gelenke und des Bewegungsapparates ermöglichen.

Es wurde nun gefunden, dass die Inhaltsstoffe des grünen Tees diese Aufgabe lösen. Aus Tariq, M. et al., "Prevention of collagen-induced arthritis in mice by a polyphenolic fraction from green tea", Proc. Natl. Acad. Sci. USA, Vol. 96, Seiten 4524-4529, (1999) ist der Inhaltsstoff Epigallocatechin Gallate (EGCG) des grünen Tees bekannt. Dieser bindet an Vimentin und blockiert Phosphorylierungen (S. Ermakova et al., The intermediate filament protein vimentin is a new target for epigallocatechin gallate; J. Biol. Chem. 280 (17), (2005), 16882-16890). Mit Epigallocatechin Gallate konnte gezeigt werden, dass die Autoimmunkomplexbildung von Autoantikörpern gegen mutiertes Vimentin konzentrationsabhängig blockiert werden kann.

In Kompetitionsexperimenten konnte gezeigt werden, dass die nicht bindende Substanz, Epicatechin (EC), die Antikörperbindung nur bis maximal 10 % blockiert. Mit Epigallocatechin Gallate (EGCG) konnte gezeigt werden, dass bei einer Konzentration von 10-100 µg/ml maximal 45 % der Antikörperbindung blockiert werden.

Wird grüner Tee jedoch mit einem geeigneten organischen oder organisch-wässrigen Lösungsmittel oder Lösungsmittelgemisch, z.B. einem Gemisch aus Dimethlysulfoxid (DMSO), Ethanol und Wasser extrahiert, kann überraschenderweise mit geringen Mengen dieses Extraktes eine nahezu vollständige Blockierung der Autoimmunkomplexbildung erzielt werden. Des Weiteren können auch Produkte verwendet werden, die aus solchen Extrakten, z.B. durch Trocknung, Lyophilisierung, Fraktonierung etc. erhältlich sind. Die Wirkung des Extraktes steht im Gegensatz zum Stand der Technik, da bisher weder für die bekannten einzelnen Substanzen des grünen Tees noch für eine Naturstoffmischung eine spezifische Blockierung von Antikörperreaktionen bekannt ist. Die Antikörperkomplexbildung konnte in Seren von Patienten mit anderen Autoimmunerkrankungen durch den erfindungsgemäßen Extrakt nicht signifikant verändert werden.

Diese Ergebnisse sind als äußerst überraschend anzusehen, da aus dem Stand der Technik bisher keine eigenständige Bindung an ein mutiertes Vimentin bekannt ist. Des Weiteren ist die Wechselwirkung der Inhaltsstoffe des grünen Tees mit Antikörpern aus dem Stand der Technik nicht bekannt.

Außerdem war bisher für EGCG nur bekannt, dass es a) eine antioxidative Wirkung hat, b) reaktive Zwischenstufen karzinogener Stoffe abfängt, c) kanzinogenaktivierende Enzyme hemmt und d) die Nitrosierung und die Zellproliferation (insbesondere von Tumorzellen) hemmt.

Ein weiterer Gegenstand der Erfindung ist schließlich die Verwendung eines oben beschriebenen Peptids oder eines oben beschriebenen Peptid-Fragments zum Auffinden von blockierenden Naturstoffen und/oder chemischen Substanzen gegen Antikörper getriebene Entzündungsreaktionen.

Darüber hinaus können auf der Basis der erfindungsgemäßen Peptide Bioassays entwickelt werden, mit denen Naturstoffbibliotheken, kombinatorische Bibliotheken und chemische Bibliotheken auf die Gegenwart von entzündungshemmenden Substanzen analysiert werden können. Unter Verwendung von krankheitsassoziierten Antikörpern können Substanzen aus den obigen Mischungen identifiziert und gereinigt werden, die das erfindungsgemäße Antigen direkt oder indirekt über Blockierung des Antikörpers blockieren. Für diese Substanzen konnte im Tierexperiment eine die Rheumatoide Arthritis verzögernde und in höheren Dosen vollständige Heilung beobachtet werden.

Des Weiteren soll die vorliegende Erfindung durch die folgenden Abbildungen und Beispiele veranschaulicht werden.

### Abbildungslegenden

**Abbildung 1****:** Vergleich der diagnostischen Spezifizität des Nachweises von RA-Autoantikörpern mit mutiertem Vimentin (●) im Vergleich zu citrullinierten Peptiden (CCP) (▲).
**Abbildung 2****:** Isolierte CD4-positive T-Zellen supprimieren die proliferative Antwort von CD4-negativen Zellen nach einer Peptidimmuntherapie.
**Abbildung 3****:** Hemmung der Reaktion von Antikörpern aus RA-Seren durch Extrakte aus grünem Tee, mutiertes Vimentin und Kombinationen davon.
**Abbildung 4****:** Selektivität von Extrakten aus grünem Tee bei der Blockierung von Autoantikörpern aus RA-Patienten.
**Abbildung 5****:** Allgemeine Darstellung eines Arthritis-Tiermodells
**Abbildungen 6-8:** Wirksamkeit von Extrakten aus grünem Tee *in vivo*

### Beispiele

### Beispiel 1: Identifizierung von mutierten Varianten des humanen Vimentins als RA-assoziierte Antigene

Es wurde ein Zellextrakt aus U937-Zellen (humane Monocytenzelllinie) hergestellt. Citrullinierte Proteine wurden aus dem Zellextrakt durch Präabsorption mit einem kommerziellen anti-citrullin-Antikörper entfernt. Eine nachfolgende Affinitätschromatographie mit anti-Vimentin Antikörper vom Huhn lieferte ein Eluat, aus dem Vimentin-Varianten isoliert werden konnten, die mit Antikörpern von Patienten mit RA kreuzreagierten. Die erhaltenen Vimentin-Varianten wurden weitergehend charakterisiert durch Reverse Phase Chromatographie, proteolytischen Verdau und Aminosäuresequenzierung. Im Ergebnis dessen konnten die nachfolgend beschriebenen Polypeptide mit den Aminosäuresequenzen SEQ ID No. 2-9 identifiziert werden.

### Beispiel 2: Nachweis von RA-assoziierten Autoantikörpern mit einem mutierten Vimentin als Nachweisantigen

Eine rekombinante, mutierte Variante von nativem Vimentin aus Beispiel 1 (SEQ ID No. 9) wurde in E. coli exprimiert und mittels Affinitätschromatographie, unter Verwendung des His-Tag, aufgereinigt. Eine 2 mg/ml Lösung des gereinigten Proteins wurden mit Entfaltungspuffer vorbehandelt (50 mM Tris, 2 M Guanidinhydrochlorid, 5 mM CaCl₂, 2 mM DTT, 0,5 mM EDTA, 5 mM Methylammoniumchlorid, pH 7,4) und für mindestens 14 Stunden bei 4 °C und danach in PBS 1:1000 verdünnt. 100 µl dieser Lösung wurden über Nacht bei 4 °C in die Kavitäten einer Mikrotiterplatte (Herstelle: COSTAR) gegeben. Nicht gebundenes Polypeptid wurde durch dreimaliges Waschen entfernt (175 µl, Puffer PBS/0,05 % Tween). Potenziell unspezifische Kreuzreaktionen wurden durch Inkubation der Kavitäten mit 150 µl einer 3 % Rinderserumalbumin (BSA)-Lösung in PBS blockiert. Die blockierte, entleerte Platte wurde bei 37 °C für 30 min getrocknet und unter Feuchtigkeitsausschluss bei 4 °C gelagert.

Derart hergestellte Mikrotiterplatten wurden zum quantitativen Nachweis von Autoantikörpern gegen mutiertes Vimentin nach dem Prinzip des indirekten Enzymimmunoassay eingesetzt. Als Vergleich wurde ein Nachweis von Autoantikörpern gegen CCP (Citrulliniertes Peptid) durchgeführt. Dazu wurden Serenproben von gesunden Probanden und/oder Patienten differenter Erkrankungen 1:100 mit 1 % BSA/PBS verdünnt und für 30 Minuten in den Kavitäten inkubiert. Durch wiederholtes Waschen mit PBS/0,05 % Tween werden nicht gebundene Serum-Antikörper entfernt. Enzym-markierte Detektionsantikörper (insbesondere Peroxidasekonjugierte Anti-human IgG Antikörper, Verdünnung 1:10000) wurden anschließend für 15 Minuten inkubiert.

Nach dem Auswaschen des überschüssigen Detektionsantikörpers wurde 100 µl einer Substratlösung (TMB (3,3',5,5'Tetramethylbenzidin)-Tablette in 10 ml 0,05 M Phosphat-Citrat-Puffer aufgelöst und 8 ml Wasserstoffperoxid kurz vor der Verwendung hinzugefügt) für 15 Minuten zugegeben. Die Zugabe von 100 µl einer 1 M HCl stoppte die Reaktion ab und das Reaktionsprodukt färbte sich gelb. Die Intensität der Gelbfärbung wurde photometrisch bei 450 nm bestimmt, wobei die Absorption direkt proportional zur gesuchten Autoantikörperkonzentration ist.

Dabei wurden folgende Messergebnisse erzielt:

| **Seren-Nummer** | **Diagnose** | **Mutiertes Vimentin** | **CCP** |
|---|---|---|---|
| | | OD bei 450 nm | [U/ml] |
| 1 | Normalserum | 0,127 | |
| 2 | Blutspender | 0,089 | 6,5 |
| 3 | Hepatitis | 0,107 | 12,5 |
| 4 | Borrelien-Arthritis | 0,167 | 17,7 |
| 5 | Rheumatoide Arthritis | 0,784 | 4,6 |
| 6 | Rheumatoide Arthritis | 0,984 | 361,4 |
| 7 | Rheumatoide Arthritis | 2,456 | 1156,5 |
| 8 | Rheumatoide Arthritis | 1,709 | 8,6 |
| 9 | Rheumatoide Arthritis | 1,342 | 1453,1 |

Alle Patientenseren mit der Diagnosestellung "Rheumatoide Arthritis" zeigten bei der Messung in einem Tecan "SPECTRA" Photometer deutlich erhöhte Absorptionswerte verglichen mit den Normalseren und Seren von Patienten, die an anderen Erkrankungen litten.

### Beispiel 3: Entwicklung eines Diagnostikums auf Basis mutierter Varianten von nativem Vimentin

Für die Entwicklung eines Diagnostikums auf der Basis eines mutierten Vimentins wurden differente mutierte Polypeptid-Varianten von nativem Vimentin (siehe Beispiel 1) kloniert und in E.coli exprimiert. Seren von einem nach den ACR- Kriterien definierten Kollektiv von Patienten (ca. 100) mit Rheumatoider Arthritis (RA) wurden verwendet, um die Polypeptid-Variante zu identifizieren, welche die höchste Sensitivität zur Detektion von Autoantikörper bei der RA aufweist. Dazu wurden analog dem Beispiel 2 die exprimierten und gereinigten Proteine in Mikrotiterplatten beschichtet und in einem klassischen ELISA-Assay die Kreuzreaktion der Autoantikörpern bei RA-Patienten analysiert.

Die in einem ersten Screening-Verfahren erhaltenen mutierten Sequenzen wurden in weiteren Ansätzen kombiniert, um die maximal mögliche Sensitivität zu erzielen. Zur Untersuchung der Spezifität der gefundenen mutierten Polypeptid-Variante als Diagnostikum für RA wurden Seren von 34 Patienten mit anderen Autoimmunerkrankungen (u.a. SLE, Sjögren Syndrom, IDDM) und Seren von 53 gesunden Menschen verwendet. Die final erhaltenen mutierten Polypeptid-Varianten wurden analog Beispiel 2 in Mikrotiterplatten beschichtet.

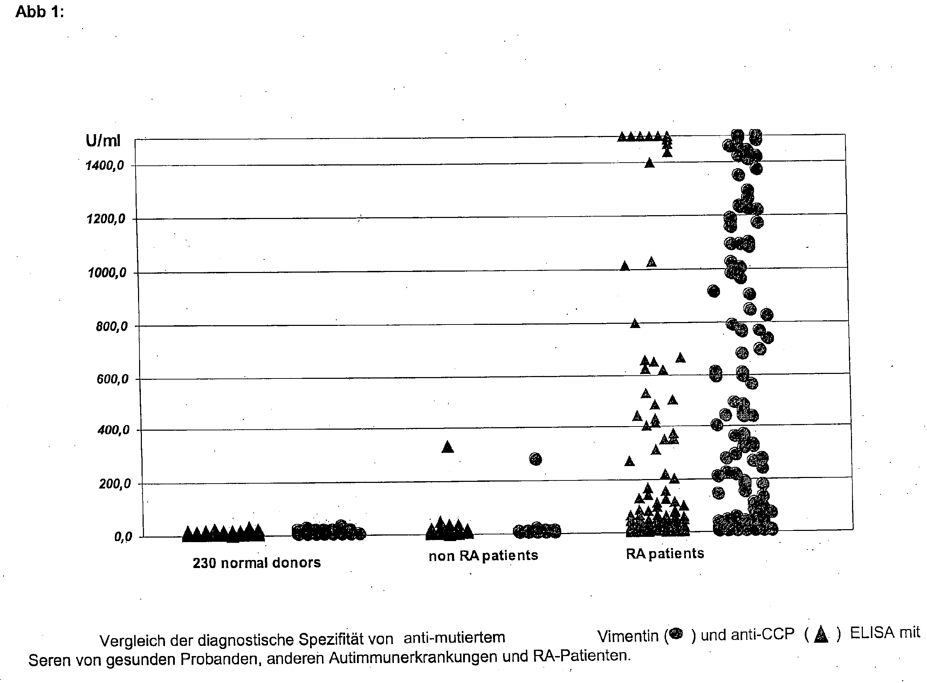

Unter Verwendung der mutierten Variante aus Beispiel 2 konnte überraschenderweise eine höhere Spezifität (> 98 %) und Sensitivität bei der Analyse der Autoantikörper von RA-Patienten und Patienten mit anderen Autoimmunerkrankungen erzielt werden als die aus dem Stand der Technik für die citrullinierten Peptide (CCP) bekannt (Abbildung 1).

### Beispiel 4: Bestimmung von Autoepitopen in mutiertem Vimentin

Anti-Vimentin-Antikörper wurden im ELISA bezüglich ihrer Bindungskapazität an überlappende biotinylierte, synthetische 17mer Peptide des mutierten Vimentins (siehe Beispiel 2), getestet. Dabei stand die Frage im Vordergrund, ob die Antikörper der Patienten mit RA gegen die gleichen B-Zell-Epitope gerichtet sind.

In der Kohorte von 102 Patienten mit RA und differentem Anti-Vimentin-Antikörper-Titer reagierten überraschenderweise 91 % der Seren mit einer der linearen Peptidsequenz aus dem aminoterminalen Bereich des Vimentins. Kreuzreaktionen gegen den carboxyterminalen Bereich oder die α-helikale, stabförmige Domäne in der Mitte konnten nicht beobachtet werden. Seren von gesunden Probanden und Patienten mit anderen Autoimmunerkrankungen (u.a. Sjögren Syndrom, Systemischer Lupus Erythematosus oder Vaskulitis) zeigten keine Reaktion gegenüber den Vimentin-Peptidsequenzen.

Eine besonders hohe Reaktivität wurde im Bereich der Aminosäuren 30-70, insbesondere im Bereich der Aminosäuren 50-65 gefunden.

### Beispiel 5: Prognosestellung bei RA-Patienten

Die RA verläuft sehr unterschiedlich und Entscheidungen das therapeutische Vorgehen sind nicht endgültig, sondern müssen fortlaufend kontrolliert und bei Bedarf angepasst werden. Deshalb wurden 21 Patienten unter medikamentöser Therapie der RA bezüglich der Reaktion gegenüber den erfindungsgemäßen Polypeptiden analysiert. In einer Follow-up Analyse (mindestens 7 Abnahmen pro Patient im Verlauf von 1-2 Jahren) wurde die Kreuzreaktion gegenüber überlappenden, biotinylierten, synthetischen Peptiden des mutierten Vimentins (siehe Beispiel 4) im ELISA charakterisiert. Dabei konnte festgestellt werden, dass beispielsweise bei RA-Patienten, die mit einem Peptid in Position 30-65 der Vimentinsequenz kreuzreagierten, eine rasche und effektive Therapie beobachtet wird, die teilweise nach 2 Jahren mit einer vollständigen Heilung ihren positiven Abschluss fand. Im Gegensatz dazu konnte bei RA-Patienten, die mit einem Peptid in Position 55-70 kreuzreagierten, in keinem der untersuchten Fälle ein Behandlungserfolg aus den Patientenunterlagen registriert werden. Gesunde Patienten zeigten gegenüber keinem der analysierten Peptide eine Kreuzreaktion.

Auf der Basis dieser Daten können die erfindungsgemäßen Polypeptide als Diagnostikum dienen, um den Verlauf und den Erfolg der Therapie von rheumatoiden Erkrankungen, insbesondere von Rheumatoider Arthritis, zu kontrollieren. Das heißt, ein quantitativer ELISA unter Verwendung der errfindungsgemäßen Peptide kann Patienten bereits zu Beginn der Therapie in medikamentöse "Responder" und "Non-responder" unterteilen.

### Beispiel 6: Therapeutische Wirksamkeit von Peptiden

Bestimmte Botenstoffe sind für die entzündlichen Prozesse in der Gelenkschleimhaut, im Knorpel und im Knochen verantwortlich. Der bekannteste Vertreter ist der Tumor Nekrose Faktor (TNF). Für die chronische Polyarthritis gibt es seit Jahren ein transgenes Mausmodell, das die anti-TNF-Therapie von Anfang an wesentlich unterstützt hat. Diese Mäuse überexprimieren humanen TNF und das reicht aus, dass sie eine schwere, chronische, destruierende Polyarthritis entwickeln.

Vor diesem Hintergrund wurden diese transgenen Mäuse entweder nur mit einer physiologischen Kochsalzlösung behandelt oder ihnen wurde eine Lösung von 1 µg eines Peptides (50-65) des mutierten Vimentins injiziert (analog Puga Yung et al., Epitope-specific immunotherapy induces immune deviation of proinflammatory T cells in rheumatoid arthritis, PNAS 2004 101: 4228-4233 und Zwerina et al., Single and combined inhibition of tumor necrosis factor, interleukin-1, and RANKL pathways in tumor necrosis factorinduced arthritis: effects on synovial inflammation, bone erosion, and cartilage destruction. Arthritis Rheum. 2004 Jan; 50(1):277-90). Im Anschluss wurde die Gelenkschwellung gemessen und die Gelenkschleimhaut, die Knochen- und die Knorpelschäden feingeweblich untersucht. Durch die Injektion eines Vimentin-Peptides wurde die Gelenkschwellung zu 41 % und die Entzündung der Gelenkschleimhaut zu 38 % gehemmt, die physiologische Kochsalzlösung zeigte keinen Einfluss. Kombinierte man allerdings mehrere Peptide des mutierten Vimentins, so ließ sich die Entzündung nahezu vollständig beherrschen.

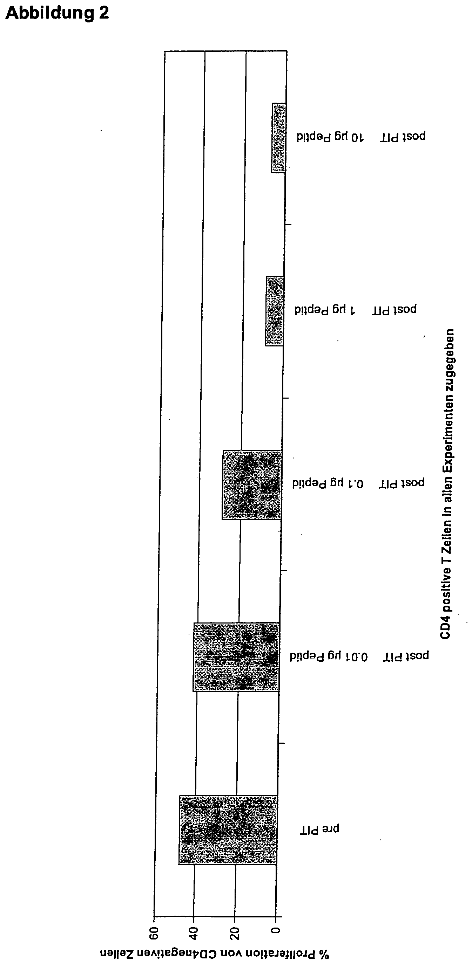

Regulatorische T-Lymphozyten gelten als aussichtsreiche Vermittler der peripheren Toleranz. Daher wurde bei diesen Tieren der Frage weiterhin nachgegangen, ob eine begrenzte Allo-Immunantwort die Bildung von Regulatorzellen antigenspezifisch induziert. Dabei wurde gefunden, dass die Mäuse nach einer solchen Behandlung Immunzellen bildeten, die erfolgreich die Teilung antigenspezifischer Immunzellen unterdrücken, wobei es sich um intrahepatische T-Zellpopulationen mit dem Phänotyp CD4⁺ CD45RC^{neg} handelt (Abbildung 2).

### Beispiel 7: Blockierung der Bildung von Autoantikörperkomplexen in vitro

Zur Blockierung der Antikörperbindung an mutiertes Vimentin wurden die folgenden Substanzen in einer Endkonzentration von 1 µg/ml einzeln oder in Kombination eingesetzt:
1. Epicatechin(EC)
2. Epigallocatechin Gallate (EGCG)
3. DMSO Extrakt aus grünem Tee
4. Mutiertes Vimentin 1,4 mg/ml
5. Mutiertes Vimentin 0,7 mg/ml
6. Citrulliniertes Vimentin 1,5 mg/ml

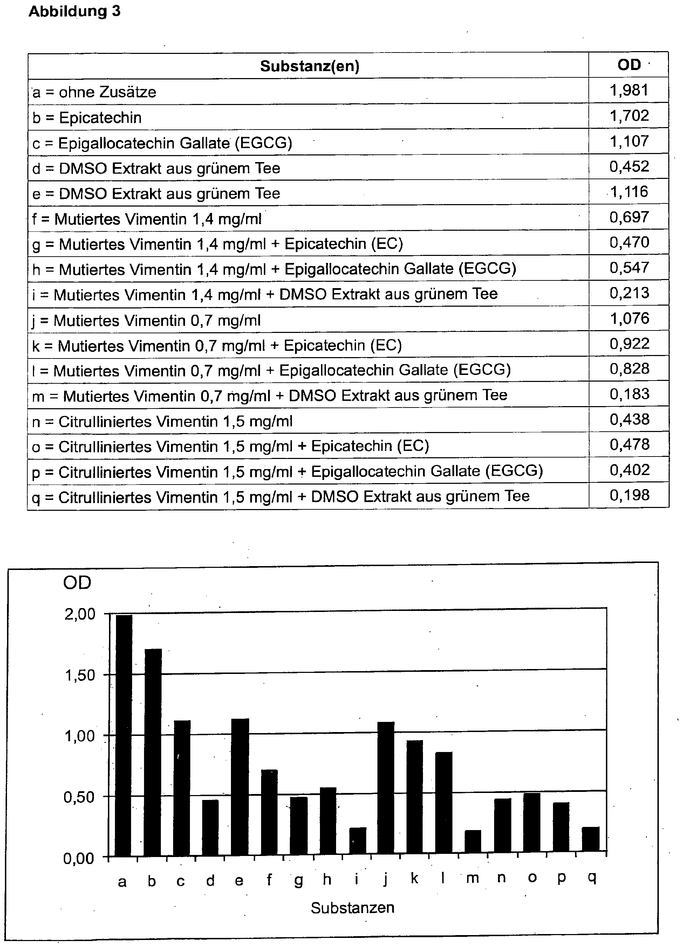

Für die Herstellung eines Extrakts aus grünem Tee wurden 5 g eines beliebigen, kommerziell erhältlichen Tees mit 10 ml 80 % DMSO überschichtet und über Nacht gerührt. Die Lösung wurde bei 13 000 rpm für 10 min zentrifugiert und als Teeextrakt definiert. Im Gegensatz zu obigen Festsubstanzen 1, 2 und 4-6 wurden vom Teeextrakt dieser Herstellung 10 µl/ml eingesetzt. Die Antikörperbindung in Gegenwart und Abwesenheit von Substanzen wurde im ELISA analysiert, wofür Mikrotiterplatten analog Beispiel 2 hergestellt wurden. Differente Seren von RA-Patienten wurden in Verdünnungen von 1:100 bis 1:400 eingesetzt. Die Blockierung der Antikörperbindung wurde induziert, indem 100 µl des verdünnten Serums für 10 min mit den angegebenen Substanzen bzw. Teelösungen präinkubiert wurde. Anschließend wurde die verbliebene Kreuzreaktion durch Übertragung der 100 µl Serum-Substanzmischung in die Kavitäten der angebenen Mikrotiterplatte analysiert. Entsprechend der Durchführung eines klaasischen ELISA's erfolgt die Detektion der gebundenen Antikörper mittels eines HRP-konjugierten Anti-human IgG's. In Abbildung 3 sind exemplarisch die erhaltenen Ergebnisse mit dem Patientenserum Nr. 400725 wiedergegeben.

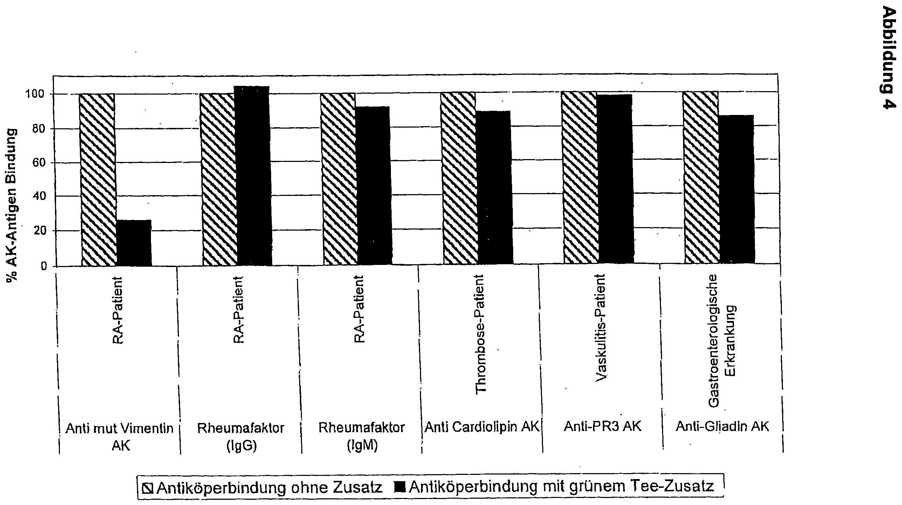

Die Spezifität der Hemmung von Autoantikörpern aus RA-Patienten (Antikörper gegen mutiertes Vimentin) durch Extrakte aus grünem Tee ist in Abbildung 4 gezeigt.

### Beispiel 8: Blockierung der Bildung von Autoantikörperkomplexen in vivo

Die orale Absorption der Catechine des grünen Tees sind bei oraler Aufnahme sehr gering, so dass bei normalem Teegenuss vermutlich nur minimale Serumkonzentrationen erreicht werden (Zhu et al., Oral absorption and bioavailability of tea catechins. Planta Medica 66 (2000) 444-7; siehe auch Schrader et al., Bioverfügbarkeit verschiedener Tee-Catechine im Plasma in Abhängigkeit von der Darreichungsform. Proc. Germ. Nutr. Soc. 3 (2001) 36).

Daher wurden die von uns hergestellten Tee-Extrakte (siehe Beispiel 7) gefriergetrocknet und mit Kakaoöl oder Lachsöl rekonstituiert. Nach oraler Applikation eines aus 5 g grünem Tee hergestellten Extraktes (5 g/kg Körpergewicht) an Ratten wurde halbstündlich Blut entnommen und mittels HPLC und GC/MS analysiert. Bei den Ratten konnte etwa 2 Stunden nach der Aufnahme eine maximale Plasmakonzentration von 75 µg//ml EC und EGCG (5-13 % der Extraktmasse) sowie etwa 245 µg/ml für EGCG (zu 50 % im Extrakt enthalten) gemessen werden. Toxische Wirkungen traten in diesen Tierexperimenten innerhalb des Untersuchungszeitraumes von 1 Monat nicht auf.

Überträgt man diese Ergebnisse auf den Menschen, so ergibt sich, dass mit dieser Darreichungsform Plasmakonzentrationen für Teeinhaltsstoffe (u.a. Catechine) erreicht werden, die eine vollständige Blockierung der Autoantikörperkomplexbildung ermöglichen könnten.

### Beispiel 9: Wirksamkeit von Extrakten des grünen Tees in vitro

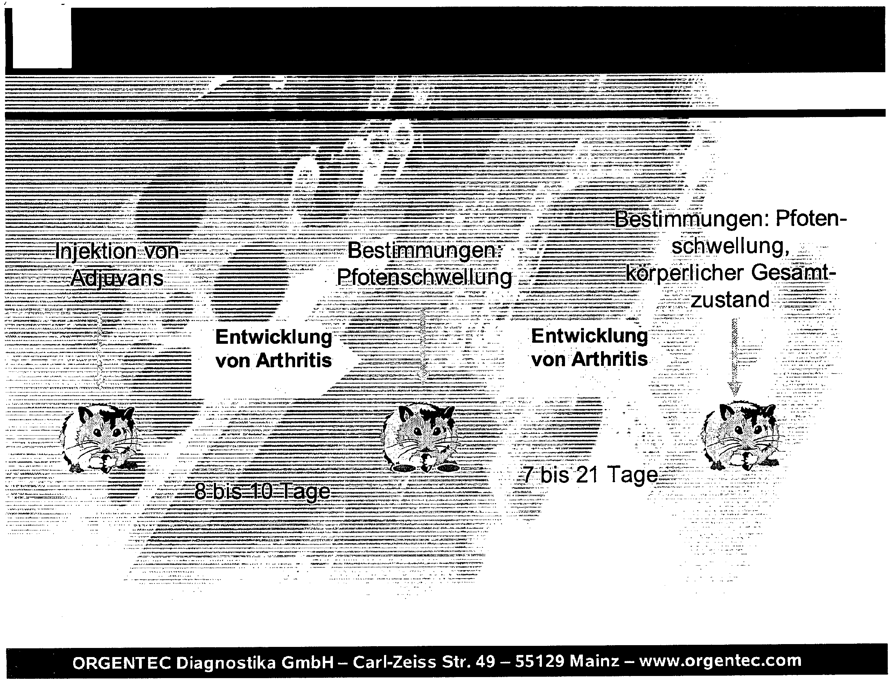

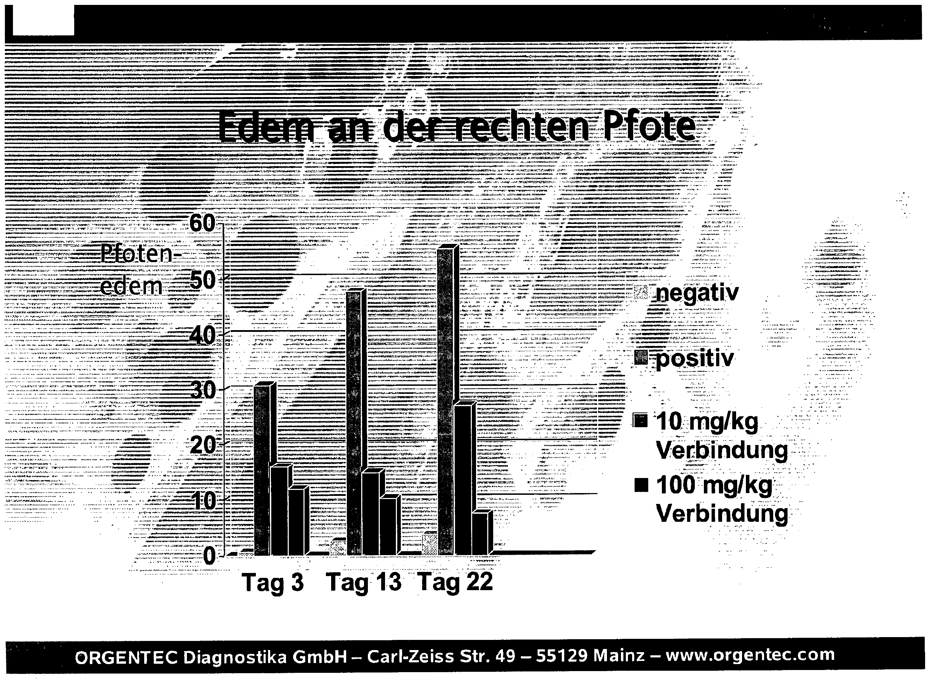

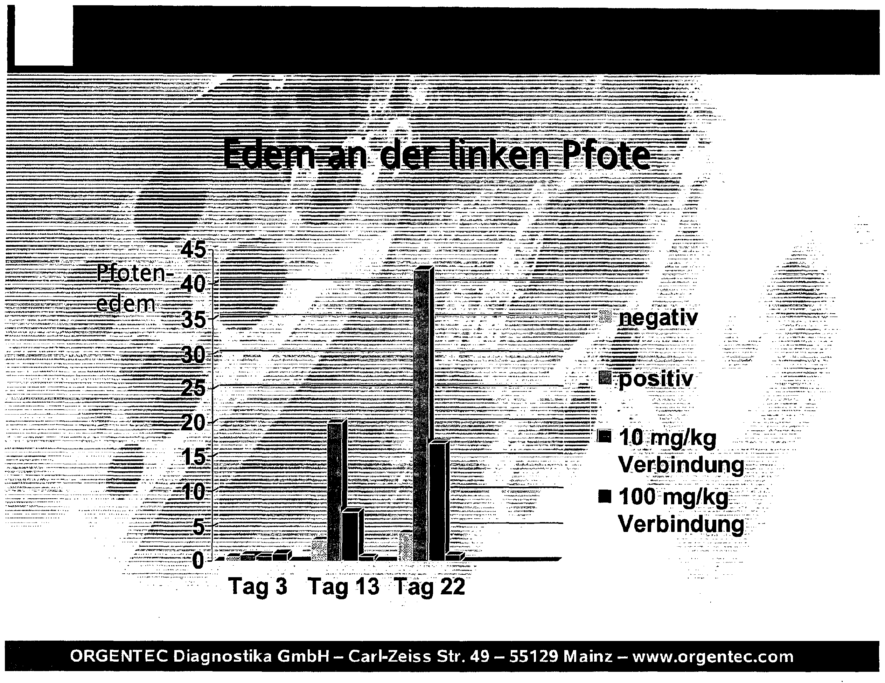

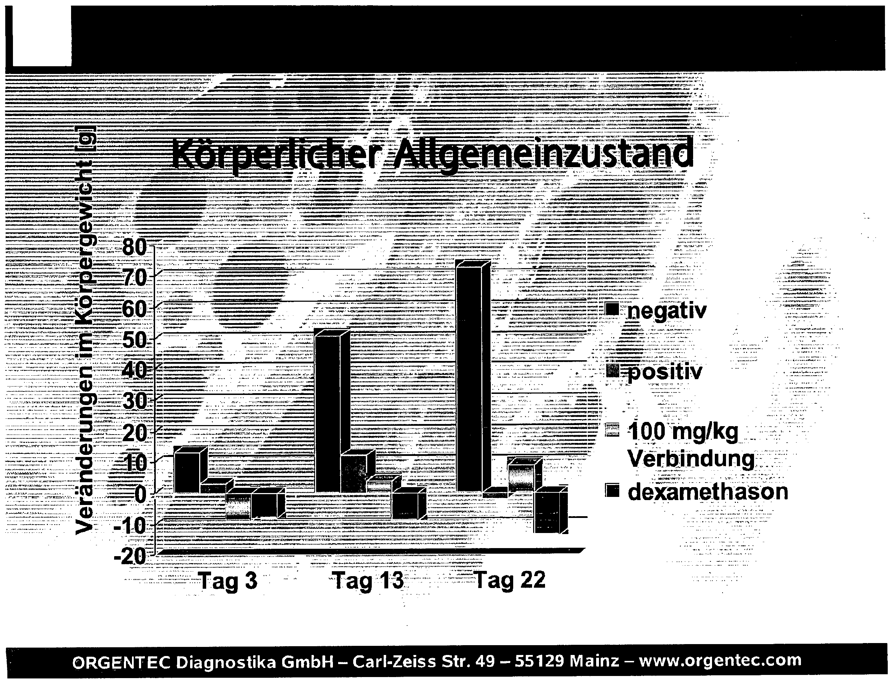

Die intraperitoneale Verabreichung eines Extrakts von grünem Tee in Konzentrationen von 10 bzw. 100 mg pro kg Körpergewicht zeigte in einem Arthritis-Tiermodell (Abbildung 5: Bestimmung der Pfotenschwellung nach Verabreichung von Adjuvans) eine deutliche positive Wirkung. Die Ergebnisse sind in den Abbildungen 6 bis 8 gezeigt.

## Patentansprüche

1. Polypeptid umfassend die Sequenz von nativem Vimentin mit der SEQ ID No. 1,
**dadurch gekennzeichnet,**
**dass** der Aminosäure-Rest in mindestens einer der Positionen 16, 17, 19, 41, 58, 59, 60, 68, 76, 140, 142, 147, 363, 406 oder 452 von SEQ ID No. 1 durch einen Arginin-Rest substituiert ist, und
**dass** gegebenenfalls der Aminosäure-Rest in mindestens einer der Positionen 3, 20, 33, 36, 37, 94, 165, 361, 399 oder 426 von SEQ ID No. 1 durch einen Leucin-Rest substituiert ist, und/oder dass gegebenenfalls der Aminosäure-Rest in mindestens einer der Positionen 21, 41, 43, 50, 54, 62, 64 oder 89 von SEQ ID No. 1 durch einen Prolin-Rest substituiert ist, und/oder dass gegebenenfalls der Aminosäure-Rest in Position 24 von SEQ ID No. 1 durch einen Threonin-Rest substituiert ist und/oder
**dass** gegebenenfalls der Aminosäure-Rest in mindestens einer der Positionen 25, 39, 42, 49, 55 oder 67 von SEQ ID No. 1 durch einen Tyrosin-Rest substituiert ist, und dass das Polypeptid eine Reaktivität gegen Rheuma-assoziierte Autoantikörper zeigt.

2. Polypeptid nach Anspruch 1,
**dadurch gekennzeichnet,**
**dass** es in mindestens zwei der Positionen 16, 17, 19, 41, 58, 59, 60, 68, 76, 140, 142, 147, 363, 406 oder 452 von SEQ ID No. 1 durch einen Arginin-Rest substituiert ist.

3. Polypeptid nach Anspruch 1,
**dadurch gekennzeichnet,**
**dass** es in mindestens zwei der Positionen 3, 20, 33, 36, 37, 94, 165, 361, 399 oder 426 von SEQ ID No. 1 durch einen Leucin-Rest substituiert ist.

4. Polypeptid nach Anspruch 1,
**dadurch gekennzeichnet,**
**dass** es in mindestens zwei der Positionen 21, 41, 43, 50, 54, 62, 64 oder 89 von SEQ ID No. 1 durch einen Prolin-Rest substituiert ist.

5. Polypeptid nach Anspruch 1,
**dadurch gekennzeichnet,**
**dass** es in mindestens zwei der Positionen 25, 39, 42, 49, 55 oder 67 von SEQ ID No. 1 durch einen Tyrosin-Rest substituiert ist.

6. Polypeptid nach Anspruch 1,
**dadurch gekennzeichnet,**
**dass** mindestens ein Arginin-Rest als Citrullin-Rest vorliegt.

7. Polypeptid nach Anspruch 6,
**dadurch gekennzeichnet,**
**dass** es in mindestens einer der Positionen 4, 12, 23, 28, 36, 45, 50, 64, 71, 100, 320, 364 oder 378 einen Citrullin-Rest aufweist.

8. Polypeptid nach Anspruch 7,
**dadurch gekennzeichnet,**
**dass** es in mindestens zwei der Positionen einen Citrullin-Rest aufweist.

9. Fragment eines Polypeptids nach Anspruch 1, bestehend aus mindestens sechs Aminosäuren von nativem Vimentin mit der SEQ ID No. 1,
**dadurch gekennzeichnet,**
**dass** der Aminosäure-Rest in mindestens einer der Positionen 16, 17, 19, 41, 58, 59, 60, 68, 76, 140, 142, 147, 363, 406 oder 452 von SEQ ID No. 1 durch einen Arginin-Rest substituiert ist, und dass es eine Reaktivität gegen Rheuma-assoziierte Autoantikörper zeigt.

10. Diagnostikum,
**dadurch gekennzeichnet,**
**dass** es ein Polypeptid oder Fragment, wie in einem der Ansprüche 1 bis 9 definiert, enthält.

11. Diagnostischer Kit zur Verwendung zum Nachweis rheumatischer Erkrankungen,
**dadurch gekennzeichnet,**
**dass** er ein Diagnostikum wie in Anspruch 10 definiert enthält.

12. Diagnostischer Kit nach Anspruch 11,
**dadurch gekennzeichnet,**
**dass** er zum Nachweis von Rheumatoider Arthritis verwendet wird.

13. Diagnostischer Kit nach Anspruch 11 oder 12,
**dadurch gekennzeichnet,**
**dass** der Träger DNA, RNA, medizinisch verträgliche Polymere, snythetische Biopolymere oder Proteine sind.

14. Verfahren zum *in vitro*-Nachweis rheumatischer Erkrankungen, bei dem die Konzentration von Autoantikörpern in einer Körperflüssigkeit bestimmt wird und gegebenenfalls zur Stellung einer Diagnose, Klassifizierung und Bewertung des Schweregrades der Erkrankung dient,
**dadurch gekennzeichnet,**
**dass** ein Diagnostikum wie in Anspruch 10 definiert oder ein diagnostischer Kit wie in einem der Ansprüche 11 bis 13 definiert, verwendet wird.

15. Verfahren nach Anspruch 14, **dadurch gekennzeichnet, dass** dieses zum Nachweis von Rheumatoider Arthritis eingesetzt wird.

16. Arzneimittel,
**dadurch gekennzeichnet,**
**dass** es ein Polypeptid oder Fragment, wie in einem der Ansprüche 1 bis 9 definiert, enthält.

17. Verwendung eines Polypeptids gemäß einer der Sequenzen SEQ ID NOs: 2-9 oder eines Fragments davon bestehend aus mindestens sechs Aminosäuren, wobei das Fragment in mindestens einer der Positionen 16, 17, 19, 41, 58, 59, 60, 68, 76, 140, 142, 147, 363, 406 oder 452 der Sequenzen SEQ ID NOs: 2-9 einen Arginin-Rest umfasst und eine Reaktivität gegen Rheuma-assoziierte Autoantikörper zeigt, zur Herstellung eines Arzneimittels zur Prophylaxe und/oder zur Behandlung von rheumatischen Erkrankungen.

18. Verwendung nach Anspruch 17,
**dadurch gekennzeichnet,**
**dass** es sich bei der rheumatischen Erkrankung um Rheumatoide Arthritis handelt.

19. Verwendung eines Polypeptids oder eines Fragments, wie in einem der Ansprüche 1 bis 9 definiert, zum Auffinden von blockierenden Naturstoffen und/oder chemischen Substanzen gegen Entzündungsreaktionen, die durch Rheuma-assoziierte Autoantikörper oder/und Autoantigene getrieben werden.

20. Verwendung nach Anspruch 17 oder 18 in Kombination mit Epigallocatechin Gallate (EGCG).

21. Verwendung nach Anspruch 17 oder 18 in Kombination mit einem Extrakt aus grünem Tee.

22. Verwendung nach Anspruch 21,
**dadurch gekennzeichnet,**
**dass** man einen organischen oder organisch-wässrigen Extrakt aus grünem Tee oder ein aus einem solchen Extrakt erhältliches Produkt einsetzt.

23. Verwendung nach Anspruch 21 oder 22,
**dadurch gekennzeichnet,**
**dass** der Extrakt als Wirkstoff Epigallocatechin Gallate (EGCG) enthält.

## Claims

1. Polypeptide comprising the sequence of native vimentin having SEQ ID No.
1, **characterised in that**
the amino acid functional group is substituted by an arginine functional group in at least one of the positions 16, 17, 19, 41, 58, 59, 60, 68, 76, 140, 142, 147, 363, 406 or 452 of SEQ ID No. 1, and
**in that** the amino acid functional group is optionally substituted by a leucine functional group in at least one of the positions 3, 20, 33, 36, 37, 94, 165, 361, 399 or 426 of SEQ ID No. 1,
and/or
**in that** the amino acid functional group is optionally substituted by a proline functional group in at least one of the positions 21, 41, 43, 50, 54, 62, 64 or 89 of SEQ ID No. 1,
and/or
**in that** the amino acid functional group is optionally substituted by a threonine functional group in position 24 of SEQ ID No. 1 and/or
**in that** the amino acid functional group is optionally substituted by a tyrosine functional group in at least one of the positions 25, 39, 42, 49, 55 or 67 of SEQ ID No. 1,
and **in that** the polypeptide exhibits reactivity against rheumatism-associated autoantibodies.

2. Polypeptide according to claim 1,
**characterised in that**
it is substituted by an arginine functional group in at least two of the positions 16, 17, 19, 41, 58, 59, 60, 68, 76, 140, 142, 147, 363, 406 or 452 of SEQ ID No. 1.

3. Polypeptide according to claim 1,
**characterised in that**
it is substituted by a leucine functional group in at least two of the positions 3, 20, 33, 36, 37, 94, 165, 361, 399 or 426 of SEQ ID No. 1.

4. Polypeptide according to claim 1,
**characterised in that**
it is substituted by a proline functional group in at least two of the positions 21, 41, 43, 50, 54, 62, 64 or 89 of SEQ ID No. 1.

5. Polypeptide according to claim 1,
**characterised in that**
it is substituted by a tyrosine functional group in at least two of the positions 25, 39, 42, 49, 55 or 67 of SEQ ID No. 1.

6. Polypeptide according to claim 1,
**characterised in that**
at least one arginine functional group is present as the citrulline functional group.

7. Polypeptide according to claim 6,
**characterised in that**
it has a citrulline functional group in at least one of the positions 4, 12, 23, 28, 36, 45, 50, 64, 71, 100, 320, 364 or 378.

8. Polypeptide according to claim 7,
**characterised in that**
it has a citrulline functional group in at least two of the positions.

9. Fragment of a polypeptide according to claim 1, consisting of at least six amino acids of native vimentin having the SEQ ID No. 1,
**characterised in that**
the amino acid functional group is substituted by an arginine functional group in at least one of the positions 16, 17, 19, 41, 58, 59, 60, 68, 76, 140, 142, 147, 363, 406 or 452 of SEQ ID No. 1, and **in that** it exhibits reactivity against rheumatism-associated autoantibodies.

10. Diagnostic agent,
**characterised in that**
it contains a polypeptide or fragment, as specified in any of claims 1 to 9.

11. Diagnostic kit for use in detecting rheumatic diseases, **characterised in that** it contains a diagnostic agent as specified in claim 10.

12. Diagnostic kit according to claim 11,
**characterised in that**
it is used to detect rheumatoid arthritis.

13. Diagnostic kit according to either claim 11 or claim 12,
**characterised in that**
the carrier is DNA, RNA, medically acceptable polymers, synthetic biopolymers or proteins.

14. Method for *in vitro* detection of rheumatic diseases, in which the concentration of autoantibodies in a bodily fluid is determined and is optionally used to make a diagnosis, classification and evaluation of the severity of the disease,
**characterised in that**
a diagnostic agent as specified in claim 10 or a diagnostic kit as specified in any of claims 11 to 13 is used.

15. Method according to claim 14, **characterised in that** said method is used to detect rheumatoid arthritis.

16. Pharmaceutical product,
**characterised in that**
it contains a polypeptide or fragment as specified in any of claims 1 to 9.

17. Use of a polypeptide according to any of the sequences of SEQ ID No. 2-9 or a fragment thereof consisting of at least six amino acids, wherein the fragment comprises an arginine functional group in at least one of the positions 16, 17, 19, 41, 58, 59, 60, 68, 76, 140, 142, 147, 363, 406 or 452 of the sequences of SEQ ID No. 2-9 and exhibits reactivity against rheumatism-associated autoantibodies, for producing a pharmaceutical product for the prevention and/or treatment of rheumatic diseases.

18. Use according to claim 17,
**characterised in that**
the rheumatic disease is rheumatoid arthritis.

19. Use of a polypeptide or a fragment, as specified in any of claims 1 to 9, for locating natural substances and/or chemical substances that block inflammatory reactions, which are driven by rheumatism-associated autoantibodies and/or autoantigens.

20. Use according to either claim 17 or claim 18 in combination with Epigallocatechin Gallate (EGCG).

21. Use according to either claim 17 or claim 18 in combination with a green tea extract.

22. Use according to claim 21,
**characterised in that**
an organic or organic-aqueous green tea extract or a product obtainable from an extract of this type is used.

23. Use according to either claim 21 or claim 22,
**characterised in that**
the extract contains Epigallocatechin Gallate (EGCG) as the active substance.

## Revendications

1. Polypeptide comprenant la séquence de la vimentine native ayant SEQ ID No. 1, **caractérisé**
**en ce que** le résidu d'acide aminé dans au moins l'une des positions 16, 17, 19, 41, 58, 59, 60, 68, 76, 140, 142, 147, 363, 406 ou 452 de SEQ ID No. 1 est substitué par un résidu d'arginine, et
**en ce que**, éventuellement, le résidu d'acide aminé dans au moins l'une des positions 3, 20, 33, 36, 37, 94, 165, 361, 399 ou 426 de SEQ ID No. 1 est substitué par un résidu de leucine,
et/ou
**en ce que**, éventuellement, le résidu d'acide aminé dans au moins l'une des positions 21, 41, 43, 50, 54, 62, 64 ou 89 de SEQ ID No. 1 est substitué par un résidu de proline,
et/ou
**en ce que**, éventuellement, le résidu d'acide aminé dans la position 24 de SEQ ID No. 1 est substitué par un résidu de thréonine et/ou
**en ce que**, éventuellement, le résidu d'acide aminé dans au moins l'une des positions 25, 39, 42, 49, 55 ou 67 de SEQ ID No. 1 est substitué par un résidu de tyrosine,
et **en ce que** le polypeptide présente une réactivité contre les autoanticorps associés au rhumatisme.

2. Polypeptide selon la revendication 1 **caractérisé en ce que**, dans au moins deux des positions 16, 17, 19, 41, 58, 59, 60, 68, 76, 140, 142, 147, 363, 406 ou 452 de SEQ ID No. 1, il est substitué par un résidu d'arginine.

3. Polypeptide selon la revendication 1 **caractérisé en ce que**, dans au moins deux des positions 3, 20, 33, 36, 37, 94, 165, 361, 399 ou 426 de SEQ ID No. 1, il est substitué par un résidu de leucine.

4. Polypeptide selon la revendication 1 **caractérisé en ce que**, dans au moins deux des positions 21, 41, 43, 50, 54, 62, 64 ou 89 de SEQ ID No. 1, il est substitué par un résidu de proline.

5. Polypeptide selon la revendication 1 **caractérisé en ce que**, dans au moins deux des positions 25, 39, 42, 49, 55 ou 67 de SEQ ID No. 1, il est substitué par un résidu de tyrosine.

6. Polypeptide selon la revendication 1 **caractérisé en ce qu'**au moins un résidu d'arginine est sous forme de résidu de citrulline.

7. Polypeptide selon la revendication 6 **caractérisé en ce que**, dans au moins l'une des positions 4, 12, 23, 28, 36, 45, 50, 64, 71, 100, 320, 364 ou 378, il présente un résidu de citrulline.

8. Polypeptide selon la revendication 7 **caractérisé en ce que**, dans au moins deux des positions, il présente un résidu de citrulline.

9. Fragment d'un polypeptide selon la revendication 1, consistant en au moins six acides aminés de la vimentine native ayant SEQ ID No. 1, **caractérisé en ce que** le résidu d'acide aminé dans au moins l'une des positions 16, 17, 19, 41, 58, 59, 60, 68, 76, 140, 142, 147, 363, 406 ou 452 de SEQ ID No. 1 est substitué par un résidu d'arginine, et **en ce qu'**il présente une réactivité contre les autoanticorps associés au rhumatisme.

10. Agent de diagnostic **caractérisé**
**en ce qu'**il contient un polypeptide ou fragment tel que défini dans l'une des revendications 1 à 9.

11. Kit de diagnostic destiné à être utilisé pour la mise en évidence de maladies rhumatismales **caractérisé**
**en ce qu'**il contient un agent de diagnostic tel que défini dans la revendication 10.

12. Kit de diagnostic selon la revendication 11 **caractérisé en ce qu'**il est utilisé pour la mise en évidence de la polyarthrite rhumatoïde.

13. Kit de diagnostic selon la revendication 11 ou 12 caractérisé en ce le vecteur est de l'ADN, de l'ARN, des polymères acceptables du point de vue médical, des biopolymères synthétiques ou des protéines.

14. Procédé pour la mise en évidence *in vitro* de maladies rhumatismales, dans lequel la concentration d'autoanticorps dans un liquide biologique est déterminée et sert éventuellement à l'établissement d'un diagnostic, d'une classification ou d'une évaluation du degré de gravité de la maladie, **caractérisé**
**en ce qu'**un agent de diagnostic tel que défini dans la revendication 10 ou un kit de diagnostic tel que défini dans l'une des revendications 11 à 13 est utilisé.

15. Procédé selon la revendication 14 **caractérisé en ce que** celui-ci est utilisé pour la mise en évidence de la polyarthrite rhumatoïde.

16. Médicament **caractérisé**
**en ce qu'**il contient un polypeptide ou fragment tel que défini dans l'une des revendications 1 à 9.

17. Utilisation d'un polypeptide selon l'une des séquences SEQ ID NO: 2-9 ou d'un fragment de celui-ci consistant en au moins six acides aminés, où le fragment dans au moins l'une des positions 16, 17, 19, 41, 58, 59, 60, 68, 76, 140, 142, 147, 363, 406 ou 452 des séquences SEQ ID NO: 2-9 comprend un résidu d'arginine, et présente une réactivité contre les autoanticorps associés au rhumatisme, pour la fabrication d'un médicament pour la prophylaxie et/ou le traitement des maladies rhumatismales.

18. Utilisation selon la revendication 17 **caractérisée en ce que** la maladie rhumatismale est la polyarthrite rhumatoïde.

19. Utilisation d'un polypeptide ou d'un fragment, tel que défini dans l'une des revendications 1 à 9, pour trouver des substances naturelles et/ou des substances chimiques bloquantes contre les réactions inflammatoires, qui sont provoquées par des autoanticorps et/ou des autoantigènes associés au rhumatisme.

20. Utilisation selon la revendication 17 ou 18 en combinaison avec des gallates d'épigallocatéchine (GEGC).

21. Utilisation selon la revendication 17 ou 18 en combinaison avec un extrait de thé vert.

22. Utilisation selon la revendication 21 **caractérisée en ce que** l'on utilise un extrait organique ou organique-aqueux de thé vert ou un produit qui peut être obtenu à partir d'un tel extrait.

23. Utilisation selon la revendication 21 ou 22 **caractérisée en ce que** l'extrait contient comme principe actif des gallates d'épigallocatéchine (GEGC).


### SEQUENCE LISTING

<110> Orgentec Diagnostika GmbH
<120> verfahren zur Diagnose der rheumatoiden Arthritis
<130> 34478PDE
<160> 9
<170> PatentIn version 3.3
<210> 1
   <211> 466
   <212> PRT
   <213> Homo sapiens
<220>
   <221> PEPTIDE
   <222> (1)..(466)
   <223> Peptidsequenz des nativen vimentins
<400> 1
   Met Ser Thr Arg Ser Val Ser Ser Ser Ser Tyr Arg Arg Met Phe Gly
   1               5                   10                  15
   Gly Pro Gly Thr Ala Ser Arg Pro Ser Ser Ser Arg Ser Tyr Val Thr
               20                  25                  30
   Thr Ser Thr Arg Thr Tyr Ser Leu Gly Ser Ala Leu Arg Pro Ser Thr
           35                  40                  45
   Ser Arg Ser Leu Tyr Ala Ser Ser Pro Gly Gly Val Tyr Ala Thr Arg
       50                  55                  60
   Ser Ser Ala Val Arg Leu Arg Ser Ser Val Pro Gly Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
   180 185 190
   Met Leu Gln Arg Glu Glu Ala Glu Asn Thr Leu Gln Ser Phe Arg Gln
   195 200 205
   Asp Val Asp Asn Ala Ser Leu Ala Arg Leu Asp Leu Glu Arg Lys Val
   210 215 220
   Glu Ser Leu Gln Glu Glu Ile Ala Phe Leu Lys Lys Leu His Glu Glu
   225 230 235 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Arg
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Gly Arg Leu Gln Asp Glu
   355 360 365
   Ile Gln Asn Met Lys Glu Glu Met Ala Arg His Leu Arg Glu Tyr Gln
   370 375 380
   Asp Leu Leu Asn Val Lys Met Ala Leu Asp Ile Glu Ile Ala Thr Tyr
   385 390 395 400
   Arg Lys Leu Leu Glu Gly Glu Glu Ser Arg Ile Ser Leu Pro Leu Pro
   405 410 415
   Asn Phe Ser Ser Leu Asn Leu Arg Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Gly Gln Val Ile Asn Glu Thr Ser Gln His His Asp Asp
       450                 455                 460
   Leu Glu
   465
<210> 2
   <211> 466
   <212> PRT
   <213> Homo sapiens
<220>
   <221> PEPTIDE
   <222> (1)..(466)
   <223> Peptidsequenzen der Mutante I
<400> 2
   Met Ser Thr Arg Ser Val Ser Ser Ser Ser Tyr Arg Arg Met Phe Arg
   1               5                   10                  15
   Arg Pro Arg Thr Ala Ser Arg Pro Ser Ser Ser Arg Ser Tyr Val Thr
               20                  25                  30
   Thr Ser Thr Arg Thr Tyr Ser Leu Arg Ser Ala Leu Arg Pro Ser Thr
           35                  40                  45
   Ser Arg Ser Leu Tyr Ala Ser Ser Pro Arg Arg Val Tyr Ala Thr Arg
       50                  55                  60
   Ser Ser Ala Val Arg Leu Arg Ser Ser Val Pro Arg Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Arg Gln Arg Lys Ser
       130                 135                 140
   Arg Leu Arg Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
   195 200 205
   Asp Val Asp Asn Ala Ser Leu Ala Arg Leu Asp Leu Glu Arg Lys Val
   210 215 220
   Glu Ser Leu Gln Glu Glu Ile Ala Phe Leu Lys Lys Leu His Glu Glu
   225 230 235 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Arg
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Arg Arg Leu Gln Asp Glu
   355 360 365
   Ile Gln Asn Met Lys Glu Glu Met Ala Arg His Leu Arg Glu Tyr Gln
   370 375 380
   Asp Leu Leu Asn Val Lys Met Ala Leu Asp Ile Glu Ile Ala Thr Tyr
   385 390 395 400
   Arg Lys Leu Leu Glu Arg Glu Glu Ser Arg Ile Ser Leu Pro Leu Pro
   405 410 415
   Asn Phe Ser Ser Leu Asn Leu Arg Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Arg Gln Val Ile Asn Glu Thr Ser Gln His His Asp Asp
   450 455 460
   Leu Glu
   465
<210> 3
   <211> 466
   <212> PRT
   <213> Homo sapiens
<220>
   <221> PEPTIDE
   <222> (1)..(466)
   <223> Peptidsequenzen der Mutante II
<400> 3
   Met Ser Leu Arg Ser Val Ser Ser Ser Ser Tyr Arg Arg Met Phe Gly
   1               5                   10                  15
   Arg Pro Gly Leu Ala Ser Arg Pro Ser Ser Ser Arg Ser Tyr Val Thr
               20                  25                  30
   Leu Ser Leu Arg Leu Tyr Ser Leu Arg Ser Ala Leu Arg Pro Ser Thr
           35                  40                  45
   Ser Arg Ser Leu Tyr Ala Ser Ser Pro Arg Gly Asp Tyr Ala Ser Gln
       50                  55                  60
   Asn Arg Trp Val Arg Leu Arg Ser Ser Val Pro Arg Val Arg Leu Leu
   65                  70                  75                  80
   Gln Asp Ser Val Asp Phe Ser Leu Ala Asp Ala Ile Asn Leu Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Arg Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Leu Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225                 230                 235                 240
   Glu Ile Gln Glu Leu Gln Ala Gln Ile Gln Glu Gln His Val Gln Ile
                   245                 250                 255
   Asp Val Asp Val Ser Lys Pro Asp Leu Thr Ala Ala Leu Arg Asp Val
               260                 265                 270
   Arg Gln Gln Tyr Glu Ser Val Ala Ala Lys Asn Leu Gln Glu Ala Glu
           275                 280                 285
   Glu Trp Tyr Lys Ser Lys Phe Ala Asp Leu Ser Glu Ala Ala Asn Arg
       290                 295                 300
   Asn Asn Asp Ala Leu Arg Gln Ala Lys Gln Glu Ser Thr Glu Tyr Arg
   305                 310                 315                 320
   Arg Gln Val Gln Ser Leu Thr Cys Glu Val Asp Ala Leu Lys Gly Thr
                   325                 330                 335
   Asn Glu Ser Leu Glu Arg Gln Met Arg Glu Met Glu Glu Asn Phe Ala
               340                 345                 350
   Val Glu Ala Ala Asn Tyr Gln Asp Leu Ile Gly Arg Leu Gln Asp Glu
           355                 360                 365
   Ile Gln Asn Met Lys Glu Glu Met Ala Arg His Leu Arg Glu Tyr Gln
       370                 375                 380
   Asp Leu Leu Asn Val Lys Met Ala Leu Asp Ile Glu Ile Ala Leu Tyr
   385                 390                 395                 400
   Arg Lys Leu Leu Glu Arg Glu Glu Ser Arg Ile Ser Leu Pro Leu Pro
                   405                 410                 415
   Asn Phe Ser Ser Leu Asn Leu Arg Glu Leu Asn Leu Asp Ser Leu Pro
               420                 425                 430
   Leu Val Asp Thr His Ser Lys Arg Thr Leu Leu Ile Lys Thr Val Glu
           435                 440                 445
   Thr Arg Asp Arg Gln Val Ile Asn Glu Thr Ser Gln His His Asp Asp
       450                 455                 460
   Leu Glu
   465
<210> 4
   <211> 466
   <212> PRT
   <213> Homo sapiens
<220>
   <221> PEPTIDE
   <222> (1)..(466)
   <223> Peptidsequenzen der Mutante III
<400> 4
   Met Ser Thr Arg Ser Val Ser Ser Ser Ser Tyr Arg Gly Met Phe Gly
   1               5                   10                  15
   Arg Pro Gly Thr Ala Ser Gly Pro Ser Ser Ser Arg Ser Tyr Val Thr
               20                  25                  30
   Thr Ser Thr Arg Thr Tyr Ser Leu Arg Ser Ala Leu Gly Pro Ser Thr
           35                  40                  45
   Ser Arg Ser Leu Tyr Ala Ser Ser Pro Gly Arg Val Tyr Ala Thr Gly
       50                  55                  60
   Ser Ser Tyr Val Arg Leu Arg Ser Ser Val Pro Arg Val Gly Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
   210 215 220
   Glu Ser Leu Gln Glu Glu Ile Ala Phe Leu Lys Lys Leu His Glu Glu
   225 230 235 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Arg
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Gly Gly Leu Gln Asp Glu
   355 360 365
   Ile Gln Asn Met Lys Glu Glu Met Ala Gly His Leu Arg Glu Tyr Gln
   370 375 380
   Asp Leu Leu Asn Val Lys Met Ala Leu Asp Ile Glu Ile Ala Thr Tyr
   385 390 395 400
   Gly Lys Leu Leu Glu Gly Glu Glu Ser Arg Ile Ser Leu Pro Leu Pro
   405 410 415
   Asn Phe Ser Ser Leu Asn Leu Gly Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Arg Gln Val Ile Asn Glu Thr Ser Gln His His Asp Asp
   450 455 460
   Leu Glu
   465
<210> 5
   <211> 466
   <212> PRT
   <213> Homo sapiens
<220>
   <221> PEPTIDE
   <222> (1)..(466)
   <223> Peptidsequenzen der Mutante IV
<400> 5
   Met Ser Thr Arg Ser Val Ser Ser Ser Ser Tyr Arg Gly Met Phe Gly
   1               5                   10                  15
   Arg Pro Gly Thr Ala Ser Arg Pro Ser Ser Ser Arg Ser Tyr Val Thr
               20                  25                  30
   Thr Ser Thr Arg Thr Tyr Ser Leu Pro Ser Ala Leu Gly Pro Ser Thr
           35                  40                  45
   Ser Pro Ser Leu Tyr Ala Ser Ser Pro Gly Gly Arg Tyr Ala Thr Pro
       50                  55                  60
   Ser Ser Tyr Arg Gly Leu Arg Ser Ser Val Pro Arg Val Gly Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225 230 235 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Arg
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Gly Arg Leu Gln Asp Glu
   355 360 365
   Ile Gln Asn Met Lys Glu Glu Met Ala Arg His Leu Arg Glu Tyr Gln
   370 375 380
   Asp Leu Leu Asn Val Lys Met Ala Leu Asp Ile Glu Ile Ala Thr Tyr
   385 390 395 400
   Arg Lys Leu Leu Glu Gly Glu Glu Ser Arg Ile Ser Leu Pro Leu Pro
   405 410 415
   Asn Phe Ser Ser Leu Asn Leu Arg Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Gly Gln Val Ile Asn Glu Thr Ser Gln His His Asp Asp
   450 455 460
   Leu Glu
   465
<210> 6
   <211> 466
   <212> PRT
   <213> Homo sapiens
<220>
   <221> PEPTIDE
   <222> (1)..(466)
   <223> Peptidsequenzen der Mutante V
<220>
   <221> MISC_FEATURE
   <222> (1)..(466)
   <223> Xaa = Citrullin
<400> 6
   Met Ser Thr Arg Ser Val Ser Ser Ser Ser Tyr Arg Arg Met Phe Arg
   1               5                   10                  15
   Arg Pro Arg Thr Pro Ser Arg Pro Ser Ser Ser Arg Ser Tyr Val Thr
               20                  25                  30
   Thr Ser Thr Arg Thr Tyr Ser Leu Arg Ser Pro Leu Arg Pro Ser Thr
           35                  40                  45
   Ser Arg Ser Leu Tyr Pro Ser Ser Pro Thr Arg Arg Tyr Pro Thr Xaa
       50                  55                  60
   Ser Ser Ala Val Arg Leu Arg Ser Ser Val Pro Arg Val Arg Leu Leu
   65                  70                  75                  80
   Gln Asp Ser Val Asp Phe Ser Leu Pro Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Arg Gln Arg Lys Ser
       130                 135                 140
   Arg Leu Arg Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
   210 215 220
   Glu Ser Leu Gln Glu Glu Ile Ala Phe Leu Lys Lys Leu His Glu Glu
   225 230 235 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Arg
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Gly Arg Leu Gln Asp Glu
   355 360 365
   Ile Gln Asn Met Lys Glu Glu Met Ala Arg His Leu Arg Glu Tyr Gln
   370 375 380
   Asp Leu Leu Asn Val Lys Met Ala Leu Asp Ile Glu Ile Ala Thr Tyr
   385 390 395 400
   Arg Lys Leu Leu Glu Gly Glu Glu Ser Arg Ile Ser Leu Pro Leu Pro
   405 410 415
   Asn Phe Ser Ser Leu Asn Leu Arg Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Gly Gln Val Ile Asn Glu Thr Ser Gln His His Asp Asp
   450 455 460
   Leu Glu
   465
<210> 7
   <211> 466
   <212> PRT
   <213> Homo sapiens
<220>
   <221> PEPTIDE
   <222> (1)..(466)
   <223> Peptidsequenzen der Mutante VI
<220>
   <221> MISC_FEATURE
   <222> (1)..(466)
   <223> Xaa = Citrullin
<400> 7
   Met Ser Thr Xaa Ser Val Ser Ser Ser Ser Tyr Xaa Arg Met Phe Gly
   1               5                   10                  15
   Gly Pro Gly Thr Ala Ser Xaa Pro Ser Ser Ser Arg Ser Tyr Val Thr
               20                  25                  30
   Thr Ser Thr Xaa Thr Tyr Ser Leu Gly Ser Ala Leu Xaa Pro Ser Thr
           35                  40                  45
   Ser Arg Ser Leu Tyr Ala Ser Ser Pro Gly Arg Val Tyr Ala Thr Xaa
       50                  55                  60
   Ser Ser Tyr Val Arg Leu Xaa Ser Ser Val Pro Gly Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
   210 215 220
   Glu Ser Leu Gln Glu Glu Ile Ala Phe Leu Lys Lys Leu His Glu Glu
   225 230 235 240
   Glu Ile Gln Glu Leu Gln Ala Gln Ile Gln Glu Gln His Val Gln Ile
   245 250 255
   Asp Val Asp Val Ser Lys Pro Asp Leu Thr Ala Ala Leu Arg Asp Val
   260 265 270
   Arg Gln Gln Tyr Glu Ser Val Ala Ala Lys Asn Leu Gln Glu Ala Glu
   275 280 285
   Glu Trp Tyr Lys Ser Lys Phe Ala Asp Leu Ser Glu Ala Ala Asn Arg
   290 295 300
   Asn Asn Asp Ala Leu Arg Gln Ala Lys Gln Glu Ser Thr Glu Tyr Xaa
   305 310 315 320
   Arg Gln Val Gln Ser Leu Thr Cys Glu Val Asp Ala Leu Lys Gly Thr
   325 330 335
   Asn Glu Ser Leu Glu Arg Gln Met Arg Glu Met Glu Glu Asn Phe Ala
   340 345 350
   Val Glu Ala Ala Asn Tyr Gln Asp Thr Ile Gly Xaa Leu Gln Asp Glu
   355 360 365
   Ile Gln Asn Met Lys Glu Glu Met Ala Xaa His Leu Arg Glu Tyr Gln
   370 375 380
   Asp Leu Leu Asn Val Lys Met Ala Leu Asp Ile Glu Ile Ala Thr Tyr
   385 390 395 400
   Arg Lys Leu Leu Glu Gly Glu Glu Ser Arg Ile Ser Leu Pro Leu Pro
   405 410 415
   Asn Phe Ser Ser Leu Asn Leu Arg Glu Thr Asn Leu Asp Ser Leu Pro
   420 425 430
   Leu Val Asp Thr His Ser Lys Arg Thr Leu Leu Ile Lys Thr Val Glu
   435 440 445
   Thr Arg Asp Gly Gln Val Ile Asn Glu Thr Ser Gln His His Asp Asp
   450 455 460
   Leu Glu
   465
<210> 8
   <211> 466
   <212> PRT
   <213> Homo sapiens
<220>
   <221> PEPTIDE
   <222> (1)..(466)
   <223> Peptidsequenzen der Mutante VII
<220>
   <221> MISC_FEATURE
   <222> (1)..(466)
   <223> Xaa = Citrullin
<400> 8
   Met Ser Thr Arg Ser Val Ser Ser Ser Ser Tyr Arg Arg Met Phe Gly
   1               5                   10                  15
   Gly Pro Gly Thr Ala Ser Xaa Thr Tyr Ser Ser Xaa Gly Tyr Val Thr
               20                  25                  30
   Leu Ser Thr Xaa Thr Tyr Tyr Leu Gly Tyr Ala Leu Xaa Pro Ser Thr
           35                  40                  45
   Tyr Xaa Ser Leu Tyr Ala Tyr Ser Pro Gly Gly Val Tyr Ala Thr Xaa
       50                  55                  60
   Gly Ser Tyr Arg Arg Leu Xaa Gly Ser Val Pro Gly Val Arg Leu Leu
   65                  70                  75                  80
   Gln Asp Ser Val Asp Phe Ser Leu Ala Asp Ala Ile Asn Thr Glu Phe
                   85                  90                  95
   Lys Asn Thr Xaa Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225                 230                 235                 240
   Glu Ile Gln Glu Leu Gln Ala Gln Ile Gln Glu Gln His Val Gln Ile
                   245                 250                 255
   Asp Val Asp Val Ser Lys Pro Asp Leu Thr Ala Ala Leu Arg Asp Val
               260                 265                 270
   Arg Gln Gln Tyr Glu Ser Val Ala Ala Lys Asn Leu Gln Glu Ala Glu
           275                 280                 285
   Glu Trp Tyr Lys Ser Lys Phe Ala Asp Leu Ser Glu Ala Ala Asn Arg
       290                 295                 300
   Asn Asn Asp Ala Leu Arg Gln Ala Lys Gln Glu Ser Thr Glu Tyr Arg
   305                 310                 315                 320
   Arg Gln Val Gln Ser Leu Thr Cys Glu Val Asp Ala Leu Lys Gly Thr
                   325                 330                 335
   Asn Glu Ser Leu Glu Arg Gln Met Arg Glu Met Glu Glu Asn Phe Ala
               340                 345                 350
   Val Glu Ala Ala Asn Tyr Gln Asp Thr Ile Gly Arg Leu Gln Asp Glu
           355                 360                 365
   Ile Gln Asn Met Lys Glu Glu Met Ala Arg His Leu Arg Glu Tyr Gln
       370                 375                 380
   Asp Leu Leu Asn Val Lys Met Ala Leu Asp Ile Glu Ile Ala Thr Tyr
   385                 390                 395                 400
   Arg Lys Leu Leu Glu Gly Glu Glu Ser Arg Ile Ser Leu Pro Leu Pro
                   405                 410                 415
   Asn Phe Ser Ser Leu Asn Leu Arg Glu Thr Asn Leu Asp Ser Leu Pro
               420                 425                 430
   Leu Val Asp Thr His Ser Lys Arg Thr Leu Leu Ile Lys Thr Val Glu
           435                 440                 445
   Thr Xaa Asp Gly Gln Val Ile Asn Glu Thr Ser Gln His His Asp Asp
       450                 455                 460
   Leu Glu
   465
<210> 9
   <211> 466
   <212> PRT
   <213> Homo sapiens
<220>
   <221> PEPTIDE
   <222> (1)..(466)
   <223> Peptidsequenzen der Mutante VIII
<400> 9
   Met Ser Thr Arg Ser Val Ser Ser Ser Ser Tyr Arg Arg Met Phe Arg
   1               5                   10                  15
   Gly Thr Gly Thr Ala Ser Arg Thr Ser Ser Ser Arg Ser Tyr Val Thr
               20                  25                  30
   Thr Ser Thr Arg Thr Tyr Ser Leu Arg Ser Ala Leu Arg Thr Ser Thr
           35                  40                  45
   Ser Arg Ser Leu Tyr Tyr Ser Ser Pro Gly Arg Val Tyr Ala Thr Arg
       50                  55                  60
   Ser Ser Tyr Val Arg Leu Arg Ser Ser Val Thr Arg Val Ser Leu Leu
   65                  70                  75                  80
   Gln Asp Ser Val Asp Phe Ser Leu Ala Asp Ala Ile Asn Thr Glu Phe
                   85                  90                  95
   Lys Asn Thr Arg Thr Asn Glu Lys Val Glu Leu Gln Glu Leu Asn Asp
               100                 105                 110
   Arg Phe Ala Asn Tyr Ile Asp Lys Val Arg Phe Leu Glu Gln Gln Asn
           115                 120                 125
   Lys Ile Leu Leu Ala Glu Leu Glu Gln Leu Lys Gly Gln Gly Lys Ser
       130                 135                 140
   Arg Leu Gly Asp Leu Tyr Glu Glu Glu Met Arg Glu Leu Arg Arg Gln
   145                 150                 155                 160
   Val Asp Gln Leu Thr Asn Asp Lys Ala Arg Val Glu Val Glu Arg Asp
                   165                 170                 175
   Asn Leu Ala Glu Asp Ile Met Arg Leu Arg Glu Lys Leu Gln Glu Glu
               180                 185                 190
   Met Leu Gln Arg Glu Glu Ala Glu Asn Thr Leu Gln Ser Phe Arg Gln
           195                 200                 205
   Asp Val Asp Asn Ala Ser Leu Ala Arg Leu Asp Leu Glu Arg Lys Val
       210                 215                 220
   Glu Ser Leu Gln Glu Glu Ile Ala Phe Leu Lys Lys Leu His Glu Glu
   225                 230                 235                 240
   Glu Ile Gln Glu Leu Gln Ala Gln Ile Gln Glu Gln His Val Gln Ile
                   245                 250                 255
   Asp Val Asp Val Ser Lys Pro Asp Leu Thr Ala Ala Leu Arg Asp Val
               260                 265                 270
   Arg Gln Gln Tyr Glu Ser Val Ala Ala Lys Asn Leu Gln Glu Ala Glu
           275                 280                 285
   Glu Trp Tyr Lys Ser Lys Phe Ala Asp Leu Ser Glu Ala Ala Asn Arg
       290                 295                 300
   Asn Asn Asp Ala Leu Arg Gln Ala Lys Gln Glu Ser Thr Glu Tyr Arg
   305                 310                 315                 320
   Arg Gln Val Gln Ser Leu Thr Cys Glu Val Asp Ala Leu Lys Gly Thr
                   325                 330                 335
   Asn Glu Ser Leu Glu Arg Gln Met Arg Glu Met Glu Glu Asn Phe Ala
               340                 345                 350
   Val Glu Ala Ala Asn Tyr Gln Asp Thr Ile Gly Arg Leu Gln Asp Glu
           355                 360                 365
   Ile Gln Asn Met Lys Glu Glu Met Ala Arg His Leu Arg Glu Tyr Gln
       370                 375                 380
   Asp Leu Leu Asn Val Lys Met Ala Leu Asp Ile Glu Ile Ala Thr Tyr
   385                 390                 395                 400
   Arg Lys Leu Leu Glu Gly Glu Glu Ser Arg Ile Ser Leu Pro Leu Pro
                   405                 410                 415
   Asn Phe Ser Ser Leu Asn Leu Arg Glu Thr Asn Leu Asp Ser Leu Pro
               420                 425                 430
   Leu Val Asp Thr His Ser Lys Arg Thr Leu Leu Ile Lys Thr Val Glu
           435                 440                 445
   Thr Arg Asp Gly Gln Val Ile Asn Glu Thr Ser Gln His His Asp Asp
       450                 455                 460
   Leu Glu
   465